Europäisches Patentamt

European Patent Office

Office européen des brevets

(11) Publication number: **0 051 873**
**B1**

(19)

# EUROPEAN PATENT SPECIFICATION

(12)

(45) Date of publication of patent specification: **27.09.89**

(21) Application number: **81109579.3**

(22) Date of filing: **09.11.81**

(51) Int. Cl.⁴: **C 12 N 15/00, C 12 P 21/02, C 07 H 21/04, C 07 C 103/52, A 61 K 45/02**

(54) Hybrid human leukocyte interferons, process for their microbial production, intermediates therefor and compositions containing them.

(30) Priority: **10.11.80 US 205579**
**23.02.81 US 237388**
**25.09.81 US 305657**

(43) Date of publication of application:
**19.05.82 Bulletin 82/20**

(45) Publication of the grant of the patent:
**27.09.89 Bulletin 89/39**

(84) Designated Contracting States:
**AT BE CH DE FR GB IT LI LU NL SE**

(56) References cited:
**EP-A-0 032 134**

**PROC. NATL. ACAD. SCI. USA, vol. 78, no. 5, May 1981. M. STREULI et al.: "Target cell specificity of two species of human interferon-alpha produced in Escherichia coli and of hybrid molecules derived from them", pages 2848-2852**

(73) Proprietor: **GENENTECH, INC.**
**460 Point San Bruno Boulevard**
**South San Francisco California 94080 (US)**

(72) Inventor: **Goeddel, David Van Norman**
**1449 Benito Avenue**
**Burlingame, California (US)**

(74) Representative: **Lederer, Franz, Dr. et al**
**Van der Werth, Lederer & Riederer**
**Patentanwälte Lucile-Grahn-Strasse 22**
**D-8000 München 80 (DE)**

(56) References cited:
**NATURE, volume 287, 2nd October 1980, no. 5781, CHESHAM (GB). D.V. GOEDDEL et al.: "Human leucocyte interferon produced by E. coli is biologically active", pages 411-416**

**Gene, 10, 1-10, 1980**
**Nature 287, 408-411, 1980**
**Science 209, 1343-1347, 1980**

Courier Press, Leamington Spa, England.

# EP 0 051 873 B1

## Description

This invention relates to the microbial production, via recombinant DNA technology, of hybrid human leukocyte interferons for use in the treatment of viral and neoplastic diseases, and to the means and end products of such production.

Background of the invention

Relatively homogeneous leukocyte interferons have been derived from normal or leukemic donors' leukocytes. These interferons are a family of proteins characterized by a potent ability to confer a virus-resistant state in their target cells. In addition, interferon can act to inhibit cell proliferation and modulate immune response. These properties have prompted the clinical use of interferon as a therapeutic agent for the treatment of viral infections and malignancies.

More recently, recombinant DNA technology has been employed to identify the nucleotide sequence of a cloned human leukocyte interferon cDNA by N. Mantei et al. (Gene 10, 1—10 [1980] and to occasion the microbial production of human leukocyte interferon A by David V. Goeddel et al. (Nature 287, 411—416 [1980]. While M. Streuli et al. (Science 209, 1343—1347 [1980]) have disclosed the existence of at least three human type leukocyte interferons and Geoffrey Allen et al. (Nature 287, 408—411 [1980]) reported on the existence of a family of structural genes for human leukocyte-type interferon, David V. Goeddel et al. have described the structure of eight different human leukocyte interferons deduced from cloned cDNAs the amino acid sequences of which exhibit in the order of 70% homology, one relative to another [Nature 290, 20—26 (1981)]. Goeddel and S. Pestka obtained genes encoding amino acid sequences of various leukocyte interferons designated, inter alia, LeIF A, B, C, D, F, G and H, respectively, from the cell line KG-1 described by Koeffler, H. P. and Golde, D. W. [Science 200, 1153—1154 (1978)]. The cell line KG-1 has been deposited with the American type culture collection, ATCC Accession Number CRL 8031. Such genes, appropriately deployed in plasmidic vehicles for bacterial expression, may be employed to transform host bacteria, preferably E. coli K-12 strain 294, American type culture collection Accession No. 31446, deposited October 28, 1978.

The workhorse of recombinant DNA technology is the plasmid, a non-chromosomal loop of double-stranded DNA found in bacteria and other microbes, oftentimes in multiple copies per cell. Included in the information encoded in the plasmid DNA is that required to reproduce the plasmid in daughter cells (i.e., a "replicon") and ordinarily, one or more selection characteristics such as, in the case of bacteria, resistance to antibiotics which permit clones of the host cell containing the plasmid of interest to be recognized and preferentially grown in selective media. The utility of plasmids lies in the fact that they can be specifically cleaved by one or another restriction endonuclease or "restriction enzyme", each of which recognizes a different site on the plasmidic DNA. Thereafter heterologous genes or gene fragments may be inserted into the plasmid by endwise joining at the cleavage site or at reconstructed ends adjacent to the cleavage site. DNA recombination is performed outside the cell, but the resulting "recombinant" plasmid can be introduced into it by a process known as transformation and large quantities of the heterologous gene-containing recombinant plasmid obtained by growing the transformant. Moreover, where the gene is properly inserted with reference to portions of the plasmid which govern the transcription and translation of the encoded DNA message, the resulting expression vehicle can be used to actually produce the polypeptide sequence for which the inserted gene codes, a process referred to as expression.

Expression is initiated in a region known as the promoter which is recognized by and bound by RNA polymerase. In some cases, as in the tryptophan or "trp" promoter preferred in the practice of the present invention, promoter regions are overlapped by "operator" regions to form a combined promoter-operator. Operators are DNA sequences which are recognized by so-called repressor proteins which serve to regulate the frequency of transcription initiation at a particular promoter. The polymerase travels along the DNA, transcribing the information contained in the coding strand from its 5' to 3' end into messenger RNA which is in turn translated into a polypeptide having the amino acid sequence for which the DNA codes. Each amino acid is encoded by a nucleotide triplet or "codon" within what may for present purposes be referred to as the "structural gene", i.e. that part which encodes the amino acid sequence of the expressed product. After binding to the promoter, the RNA polymerase first transcribes nucleotides encoding a ribosome binding site, then a translation initiation or "start" signal (ordinarily ATG, which in the resulting messenger RNA becomes AUG), then the nucleotide codons within the structural gene itself. So-called stop codons are transcribed at the end of the structural gene whereafter the polymerase may form an additional sequence of messenger RNA which, because of the presence of the stop signal, will remain untranslated by the ribosomes. Ribosomes bind to the binding site provided on the messenger RNA, in bacteria ordinarily as the mRNA is being formed, and themselves produce the encoded polypeptide, beginning at the translation start signal and ending at the previously mentioned stop signal. The desired product is produced if the sequences encoding the ribosome binding site are positioned properly with respect to the AUG initiator codon and if all remaining codons follow the initiator codon in phase. The resulting product may be obtained by lysing the host cell and recovering the product by appropriate purification from other bacterial protein.

Brief summary of the invention

Nucleotide sequence studies of genes encoding the various leukocyte interferons reveals a degree of

2

commonality amongst various of them with regard to the presence and location of cleavage sites recognized by particular restriction endonucleases. According to the present invention, advantage is taken of this commonality to form, by DNA recombination, novel hybrid genes useful in the microbial production of hybrid leukocyte interferons which exhibit enhanced antiviral activity relative to those encoded by the parental genes.

The present invention relates specifically to antiviral polypeptides of approximately 165—166 amino acids which are characterized in that they consist of a discrete amino acid subsequence of human leukocyte interferon A and of human leukocyte interferon B or D, the overall amino acid sequence of said polypeptides differing from that of naturally occurring leukocyte interferons and to pharmaceutical compositions containing such a polypeptide and a carrier material.

In a preferred embodiment of the present invention the N-terminus of the hybrid polypeptides consists of amino acids 1—92 or 1—63 of LeIF-D or of amino acids 1—91 or 1—62 of LeIF-A and the carboxy terminus consists of amino acids 92—165 or 63—165 of LeIF-A or of amino acids 93—166 or 64—166 of LeIF-D or of amino acids 93—166 of LeIF-B.

The present invention also relates to the double stranded DNA sequences encoding the above-mentioned hybrid leukocyte interferons, to corresponding replicable plasmidic expression vehicles which are capable in a transformant bacterium of expressing these interferons and of said transformant bacteria.

Finally, the present invention relates to the method of forming a double stranded DNA comprising a sequence encoding one of the above-mentioned polypeptides as well as to the formation of these polypeptides by their expression in a bacterial transformant.

The parental leukocyte interferon genes, encoding the family of leukocyte interferon proteins contemplated herein, exhibit natural allelic variations from individual to individual. These variations may be demonstrated by (an) amino acid difference(s) in the overall protein sequence or by deletions, substitutions, insertions, inversions or additions of (an) amino acid(s) in said sequence. For each parental leukocyte interferon hereof, labelled LeIF A, LeIF B and LeIF D, such allelic variations are included within the scope of the label or term defining such, and thus, this invention.

The manner in which these and other objects and advantages of the invention are obtained will become further apparent from the detailed description which follows and from the accompanying drawings in which:

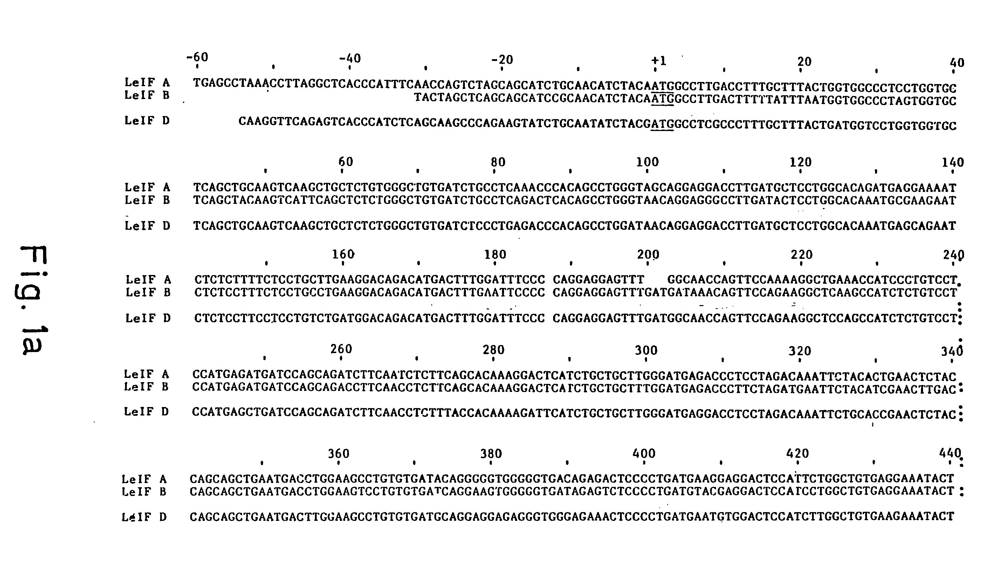

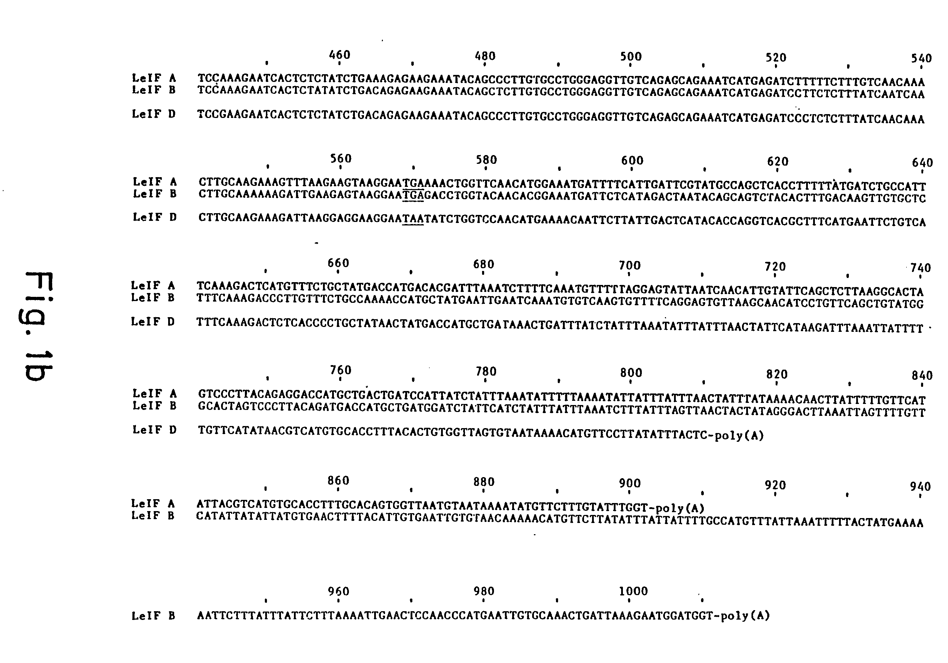

Figures 1(a, b) depicts nucleotide sequences of the coding regions of 3 leukocyte interferon ("LeIF") complementary DNA ("cDNA") clones A, B and D. The ATG translational initiation codon and the termination triplet for each LeIF is underlined.

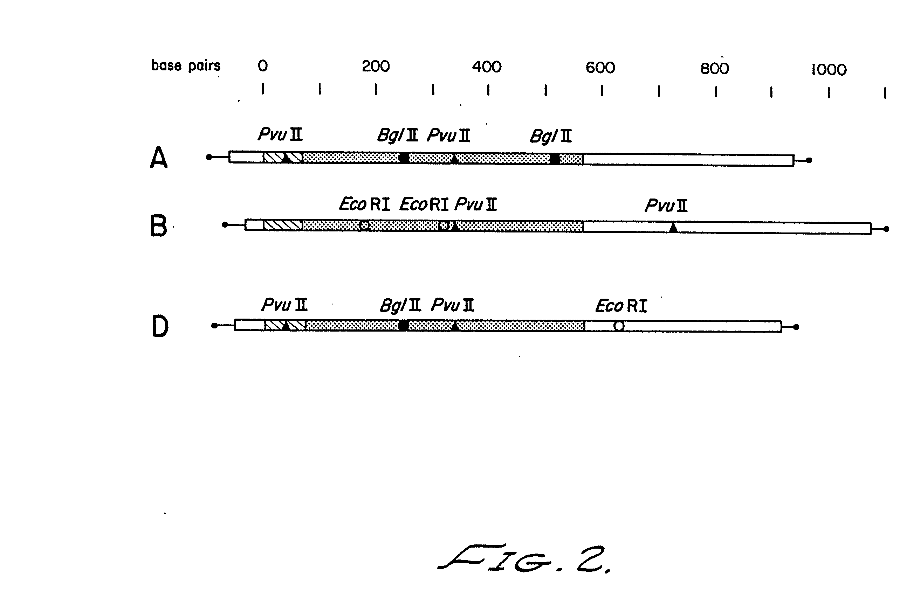

Figure 2 depicts restriction endonuclease maps of three types of LeIF cloned cDNAs (A, B and D). Plasmids containing the clones were constructed by the dC:dG tailing method [Goeddel, D. V. et al. Nature 287, 411—416 (1980)]. Therefore the cDNA inserts can be excised using Pst I, i.e., each end of each insert is a Pst I restriction endonuclease cleavage site. The lines at the end of each cDNA insert represent the flanking homopolymeric dC:dG tails. The positions of Pvu II, Eco RI and Bgl II restriction sites are indicated. Shaded regions of the Figure represent the coding sequences of mature LeIFs; the cross-hatched regions indicate signal peptide coding sequences; and the open regions show 3′ and 5′ noncoding sequences;

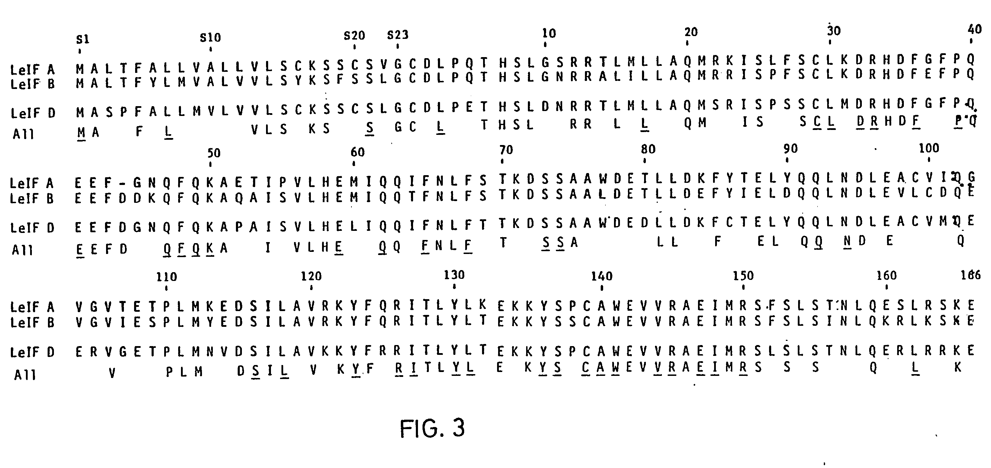

Figure 3 is a comparison of the three LeIF protein sequences predicted from the nucleotide sequences. The one letter abbreviations recommended by the IUPAC-IUB Commission on Biochemical Nomenclature are used: A, alanine; C, cysteine; D, aspartic acid; E, glutamic acid; F, phenylalanine; G, glycine; H, histidine; I, isoleucine; K, lysine; L, leucine; M, methionine; N, asparagine; P, proline, Q, glutamine; R, arginine; S, serine; T, threonine; V, valine; W, tryptophan; and Y, tyrosine. The numbers refer to amino acid position (S refers to signal peptide). The dash in the 165 amino acid LeIF A sequence at position 44 is introduced to align the LeIF A sequence with the 166 amino acid sequences of the other LeIFs. Amino acids common to all LeIFs (marked "A11") are also shown. The underlined residues are amino acids which are also present in human fibroblast interferon.

With reference to Figure 1, nucleotides +1 to 69 correspond to S1 to S23 amino acids of Figure 3. Codon TGT (nucleotides 70 to 72) of Figure 1 corresponds to cysteine (C, amino acid 1) of Figure 3. In Figure 3, the Pvu II restriction endonuclease cleavage site occurs between the codons for amino acids 92 and 93 in LeIF A, B and D, i.e., between nucleotides 346 and 347, of Figure 1.

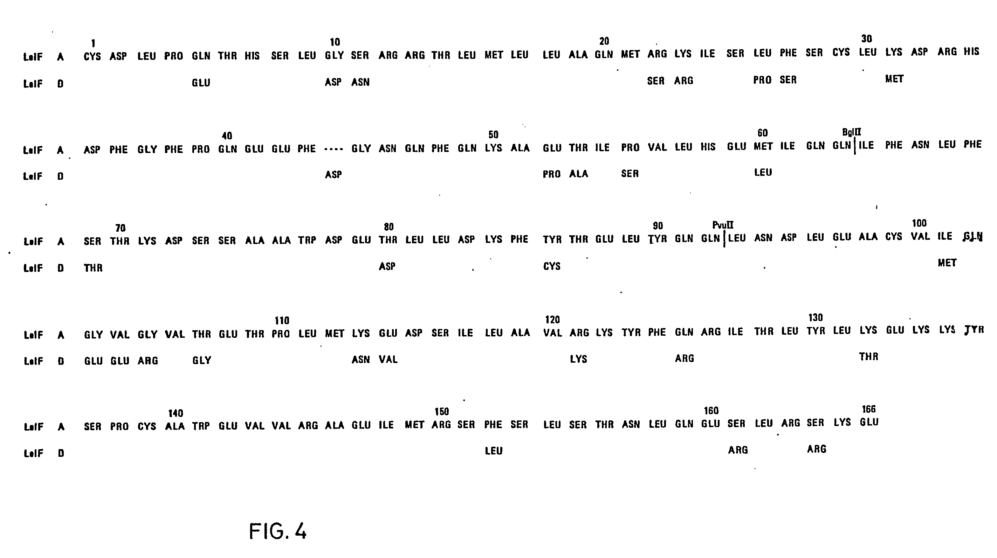

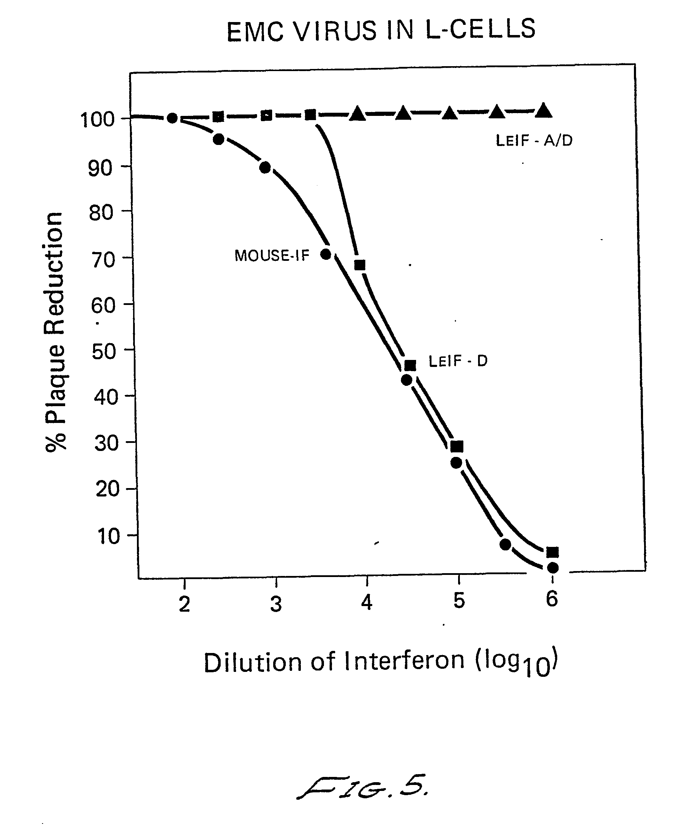

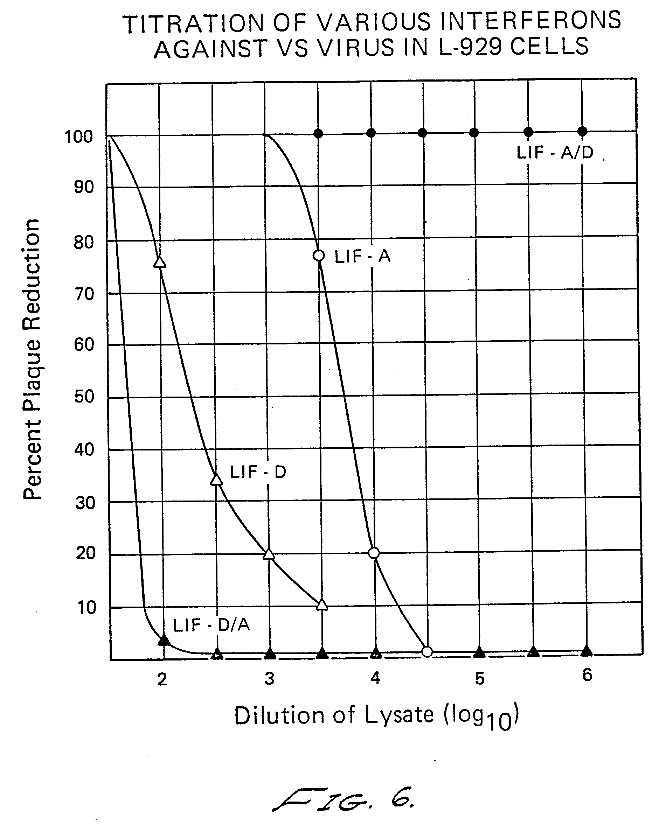

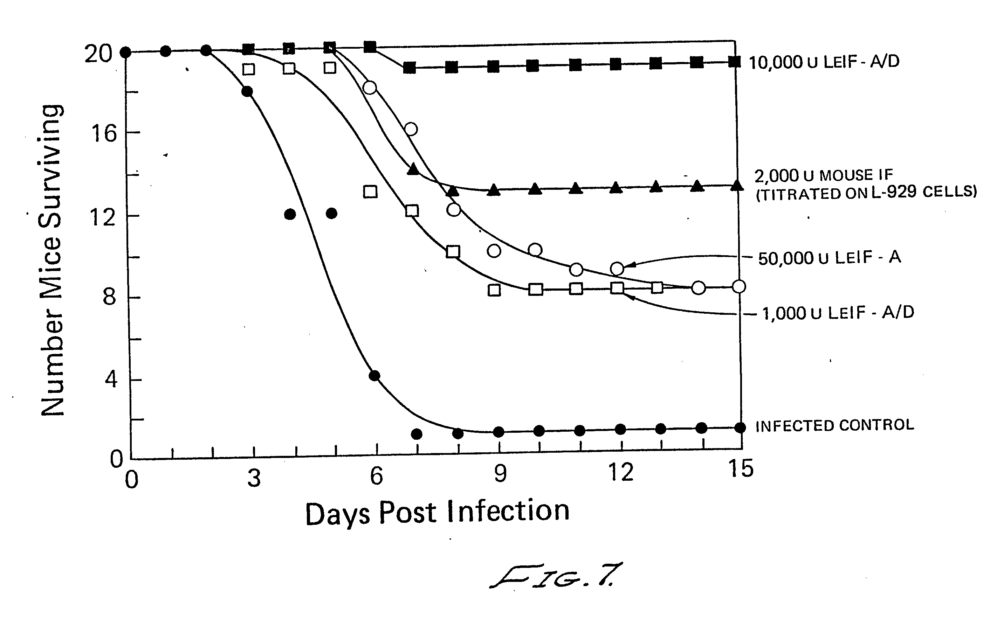

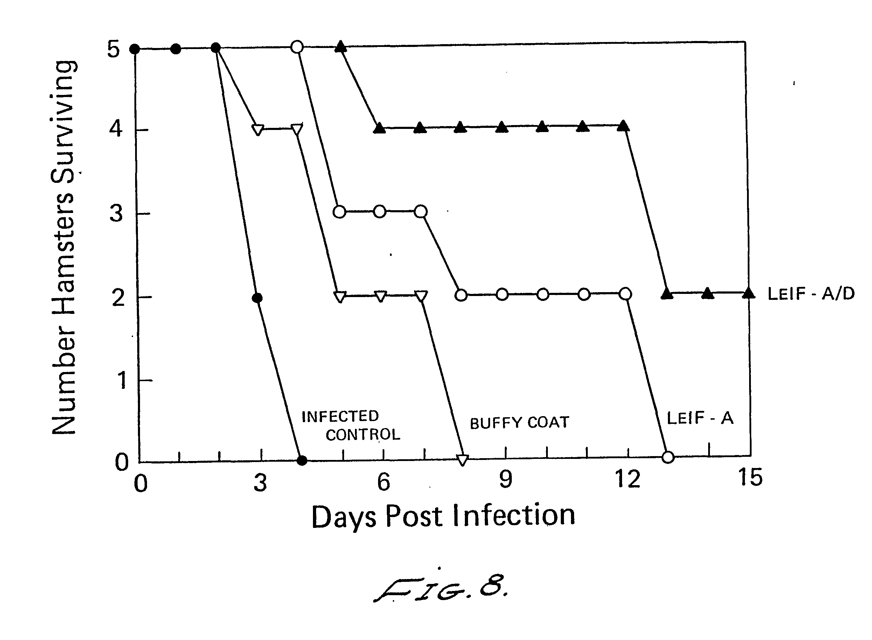

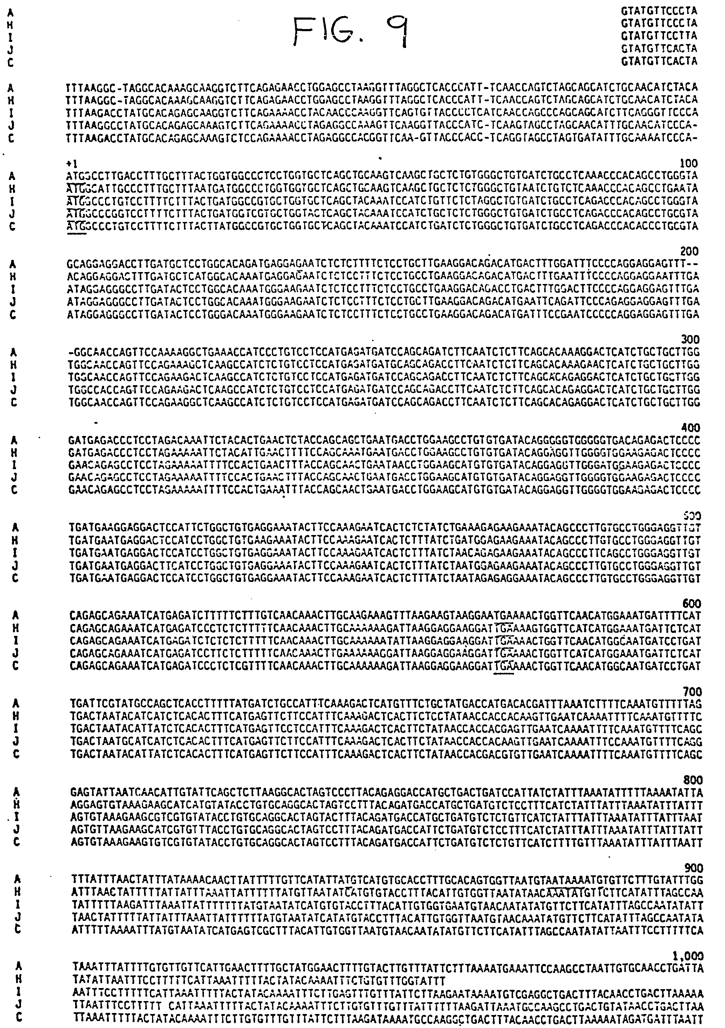

Figure 4 compares the amino acid sequences of mature leukocyte interferons A and D, a deletion of amino acid 44 in LeIF A being indicated by dashes. Only those LeIF D amino acids which differ from corresponding amino acids of LeIF A are depicted: the amino acid sequence of LeIF D is otherwise identical to LeIF A. Figure 4 also indicates the relative position on the corresponding gene of Bgl II and Pvu II restriction endonuclease cleavage sites employed in forming preferred hybrid leukocyte genes of the invention.

Figures 5 and 6 illustrate the results of comparative testing of a preferred hybrid leukocyte interferon of the invention ("LeIF-A/D") for activity against encephalomyocarditis virus ("EMC") and vesicular stomatitis virus ("VSV"), respectively in mouse cells.

Figures 7 and 8 depict the results of comparative testing involving LeIF-A/D and other interferons against EMC virus infections in, respectively, mice and hamsters. The data in Figure 7 results from treatments i.p. 3 hrs before infection. Dosages of LeIF-A/D and LeIF-A are as titrated on WISH cells.

Description of the preferred embodiments

Microorganisms employed

The work described involved use of two microorganisms: E. coli x1776, as described in U.S. Patent 4190495, and E. coli K-12 strain 294 (end A, thi⁻, hsr⁻, hsm$_k^+$), as described in British Patent Publication No. 2055382A. Each has been deposited with the American Type Culture Collection, ATCC Accession Nos. 31537 and 31446, deposited July 3, 1979 and October 28, 1978, respectively. All recombinant DNA work was performed in compliance with applicable guidelines of the National Institutes of Health.

The invention, in its most preferred embodiments, is described with reference to E. coli, including not only strains E. coli x 1776 and E. coli K-12 strain 294, defined above, but also other known E. coli strains such as E. coli B, or other microbial strains many of which are deposited and (potentially) available from recognized microorganism depository institutions, such as the American Type Culture Collection, ATCC. (See the ATCC catalogue listing and also German Offenlegungsschrift 2644432). These other microorganisms include, for example, Bacilli such as Bacillus subtilis and other enterobacteriaceae among which can be mentioned as examples Salmonella typhimurium and Serratia marcescens utilizing plasmids that can replicate and express heterologous gene sequences therein. Yeast, such as Saccharomyces cerevisiae, may also be employed to advantage as host organism in the preparation of the interferon proteins hereof by expression of genes coding therefor under the control of a yeast promoter.

The LeIF hybrids are prepared, in accordance with the present invention, by taking advantage of restriction endonuclease cleavage sites commonly located within the individual parental genes and each end thereof in conjunction with the same sites in carrier expression plasmids (vectors). For example, the large (~3900 bp) fragment of a Xba I to Pst I digest of the pLeIF A trp 25 expression plasmid can be ligated with Xba I to Pvu II and Pvu II to Pst I digest fragments of the various LeIF parental genes to provide expression plasmids operable to obtain the corresponding hybrid LeIF.

Each LeIF expression plasmid was constructed independently with digest fragments isolated from various LeIF plasmids, e.g., pLeIF A trp 25, pLeIF B trp 7, pLeIF D trp 11, etc. whose construction is described in Nature 287, 411 (1980) or from pBR322, whose construction is described in Gene 2, 95 (1977). In these plasmids, "trp" designates a tryptophan promoter—operator system most preferred for bacterial expression, as described in published European Patent Application No. 36776.

Direct expression of a first mature leukocyte interferon

The procedure followed to express Le-IF A directly as a mature interferon polypeptide involved the combination of synthetic (N-terminal) and complementary DNAs.

A Sau 3a restriction endonuclease site is conveniently located between codons 1 and 2 of Le-IF A. Two synthetic deoxyoligonucleotides were designed which incorporate an ATG translational initiation codon, restore the codon for amino acid 1 (cysteine), and create an Eco RI sticky end. These oligomers were ligated to a 34 b.p. Sau 3a-Ava II fragment of pL31. The resulting 45 b.p. product was ligated to two additional DNA fragments to construct an 865 base pair synthetic-natural hybrid gene which codes for Le-IF A and which is bounded by Eco RI and Pst I restriction sites. This gene was inserted into pBR322 between the Eco RI and Pst I sites to give the plasmid pLe-IF A1.

Plasmid pGM1 carries the E. coli tryptophan operon containing the deletion ΔLE1413 [G. F. Miozzari et al., J. Bacteriology 133, 1457—1466 (1978)] and hence expresses a fusion protein comprising the first 6 amino acids of the trp leader and approximately the last third of the trp E polypeptide (hereinafter referred to in conjunction as LE'), as well as the trp D polypeptide in its entirety, all under the control of the trp promoter-operator system. The plasmid, 20 µg, was digested with the restriction enzyme Pvu II which cleaves the plasmid at five sites. The gene fragments were next combined with EcoRI linkers (consisting of a self complementary oligonucleotide of the sequence: pCATGAATTCATG) providing an EcoRI cleavage site for a later cloning into a plasmid containing an EcoRI site. The 20 µg of DNA fragments obtained from pGM1 were treated with 10 units T₄ DNA ligase in the presence of 200 pico moles of the 5'-phosphorylated synthetic oligonucleotide pCATGAATTCATG and in 20 µl T₄ DNA ligase buffer (20 mM tris, pH 7.6, 0.5 mM ATP, 10 mM MgCl₂, 5 mM dithiothreitol) at 4°C overnight. The solution was then heated 10 minutes at 70°C to halt ligation. The linkers were cleaved by EcoRI digestion and the fragments, now with EcoRI ends were separated using 5 percent polyacrylamide gel electrophoresis (hereinafter "PAGE") and the three largest fragments isolated from the gel by first staining with ethidium bromide, locating the fragments with ultraviolet light, and cutting from the gel the portions of interest. Each gel fragment, with 300 microliters 0.1×TBE, was placed in a dialysis bag and subjected to electrophoresis at 100 v for one hour in 0.1×TBE buffer (TBE buffer contains: 10.8 gm tris base, 5.5 gm boric acid, 0.09 gm Na₂EDTA in 1 liter H₂O). The aqueous solution was collected from the dialysis bag, phenol extracted, chloroform extracted and made 0.2 M sodium chloride, and the DNA recovered in water after ethanol precipitation. The trp promoter-operator-containing gene with EcoRI sticky ends was identified in the procedure next described, which entails the insertion of fragments into a tetracycline sensitive plasmid which, upon promoter-operator insertion, becomes tetracycline resistant.

Plasmid pBRH1 (R. I. Rodriguez, et al., Nucleic Acids Research 6, 3267—3287 [1979]) expresses ampicillin resistance and contains the gene for tetracycline resistance but, there being no associated promoter, does not express that resistance. The plasmid is accordingly tetracycline sensitive. By introducing a promoter-operator system in the EcoRI site, the plasmid can be made tetracycline resistant.

4

pBRH1 was digested with EcoRI and the enzyme removed by phenol extraction followed by chloroform extraction and recovered in water after ethanol precipitation. The resulting DNA molecule was, in separate reaction mixtures, combined with each of the three DNA fragments obtained above and ligated with $T_4$ DNA ligase as previously described. The DNA present in the reaction mixture was used to transform competent E. coli K-12 strain 294 (K. Backman et al., Proc. Natl. Acad. Sci. USA 73, 4174—4198 [1976]) by standard techniques (V. Hershfield et al., Proc. Natl. Acad. Sci. USA 71, 3455—3459 [1974]) and the bacteria plated on LB plates containing 20 µg/ml ampicillin and 5 µg/ml tetracycline. Several tetracycline-resistant colonies were selected, plasmid DNA isolated and the presence of the desired fragment confirmed by restriction enzyme analysis. The resulting plasmid is designated pBRHtrp.

An EcoRI and BamHI digestion product of the viral genome of hepatitis B was obtained by conventional means and cloned into the EcoRI and BamHI sites of plasmid pGH6 (D. V. Goeddel et al., Nature 281, 544 [1979])) to form the plasmid pHS32. Plasmid pHS32 was cleaved with Xbal, phenol extracted, chloroform extracted and ethanol precipitated. It was then treated with-1 µl E. coli polymerase I, Klenow fragment (Boehringer-Mannheim) in 30 µl polymerase buffer (50 mM potassium phosphate pH 7.4, 7 mM $MgCl_2$, 1 mM β-mercaptoethanol) containing 0.1 mM dTTP and 0.1 mM dCTP for 30 minutes at 0°C then 2 hr at 37°C. This treatment causes 2 of the 4 nucleotides complementary to the 5' protruding end of the Xbal cleavage site to be filled in:

$$5' \quad CTAGA— \qquad 5' \quad CTAGA—$$
$$\rightarrow$$
$$3' \quad T— \qquad 3' \quad TCT—$$

Two nucleotides, dC and dT, were incorporated giving an end with two 5' protruding nucleotides. This linear residue of plasmid pHS32 (after phenol and chloroform extraction and recovery in water after ethanol precipitation) was cleaved with EcoRI. The large plasmid fragment was separated from the smaller EcoRI-Xbal fragment by PAGE and isolated after electroelution. This DNA fragment from pHS32 (0.2 µg), was ligated, under conditions similar to those described above, to the EcoRI-Taq I fragment of the tryptophan operon (~0.01 µg), derived from pBRHtrp.

In the process of ligating the fragment from pHS32 to the Eco RI-Taq I fragment, as described above, the Taq I protruding end is ligated to the Xbal remaining protruding and even though it is not completely Watson-Crick base-paired:

$$—T \qquad CTAGA— \qquad —TCTAGA—$$
$$+ \qquad \rightarrow$$
$$—AGC \qquad TCT— \qquad —AGCTCT—$$

A portion of this ligation reaction mixture was transformed into E. coli 294 cells, heat treated and plated on LB plates containing ampicillin. Twenty-four colonies were selected, grown in 3 ml LB media, and plasmid isolated. Six of these were found to have the Xbal site regenerated via E. coli catalyzed DNA repair and replication:

$$—TCTAGA— \qquad —TCTAGA—$$
$$\rightarrow$$
$$—AGCTCT— \qquad —AGATCT—$$

These plasmids were also found to cleave both with EcoRI and Hpal and to give the expected restriction fragments. One plasmid, designated pTrp 14, was used for expression of heterologous polypeptides, as next discussed.

The plasmid pHGH 107 (D. V. Goeddel et al, Nature, 281, 544, [1979]) contains a gene for human growth hormone made up of 23 amino acid codons produced from synthetic DNA fragments and 163 amino acid codons obtained from complementary DNA produced via reverse transcription of human growth hormone messenger RNA. This gene, though it lacks the codons of the "pre" sequence of human growth hormone, does contain an ATG translation initiation codon. The gene was isolated from 10 µg pHGH 107 after treatment with EcoRI followed by E. coli polymerase I Klenow fragment and dTTP and dATP as described above. Following phenol and chloroform extraction and ethanol precipitation the plasmid was treated with BamHI.

The human growth hormone ("HGH") gene-containing fragment was isolated by PAGE followed by electroelution. The resulting DNA fragment also contains the first 350 nucleotides of the tetracycline resistance structural gene, but lacks the tetracycline promoter-operator system so that, when subsequently cloned into an expression plasmid, plasmids containing the insert can be located by the restoration of tetracycline resistance. Because the EcoRI end of the fragment has been filled in by the Klenow polymerase I procedure, the fragment has one blunt and one sticky end, ensuring proper orientation when later inserted into an expression plasmid.

The expression plasmid pTrp14 was next prepared to receive the HGH gene-containing fragment prepared above. Thus, pTrp14 was Xbal digested and the resulting sticky ends filled in with the Klenow polymerase I procedure employing dATP, dTTP, dGTP and dCTP. After phenol and chloroform extraction and ethanol precipitation the resulting DNA was treated with BamHI and the resulting large plasmid fragment isolated by PAGE and electroelution. The pTrp14-derived fragment had one blunt and one sticky end, permitting recombination in proper orientation with the HGH gene containing fragment previously described.

The HGH gene fragment and the pTrp14 ΔXba-BamHI fragment were combined and ligated together under conditions similar to those described above. The filled in Xbal and EcoRI ends ligated together by blunt end ligation to recreate both the Xbal and the EcoRI site:

| Xbal filled in | EcoRI filled in | HGH gene initiation |
|---|---|---|
| —TCTAG | AATTCTATG— | —TCTAGAATTCTATG— |
| + | → | |
| —AGATC | TTAAGATAC— | —AGATCTTAAGATAC— |
| | | Xbal     EcoRI |

This construction also recreates the tetracycline resistance gene. Since the plasmid pHGH 107 expresses tetracycline resistance from a promoter lying upstream from the HGH gene (the lac promoter), this construction, designated pHGH 207, permits expression of the gene for tetracycline resistance under the control of the tryptophan promoter-operator. Thus the ligation mixture was transformed into E. coli 294 and colonies selected on LB plates containing 5 µg/ml tetracycline.

Plasmid pHGH207 was Eco RI digested and the trp promoter containing a 300 b.p. Eco RI fragment recovered by PAGE followed by electroelution. The 300 b.p. Eco RI fragment contains the E. coli trp promoter, operator, and trp leader ribosome binding site but lacks an ATG sequence for initiation of translation. This DNA fragment was cloned into the Eco RI site of pLe-IF A.

The trp fragment just referred to is an analog of the E. coli tryptophan operon from which the so-called trp attenuator has been deleted, to controllably heighten expression levels. Expression plasmids containing the modified trp regulon can be grown to predetermined levels in nutrient media containing additive tryptophan in quantities sufficient to repress the promoter-operator system, then be deprived of tryptophan so as to derepress the system and occasion the expression of the intended product.

More particularly, 250 µg of plasmid pL31 were digested with Pst I and the 1000 b.p. insert isolated by gel electrophoresis on a 6% polyacrylamide gel. Approximately 40 µg of insert was electroeluted from the gel and divided into 3 aliquots for further digestion: a) A 16 µg sample of this fragment was partially digested with 40 units of Bgl II for 45' at 37°C and the reaction mixture purified on a 6% polyacrylamide gel. Approximately 2 µg of the desired 670 b.p. fragment were recovered. b) Another sample (8 µg) of the 1000 b.p. Pst I insert was restricted with Ava II and Bgl II. One µg of the indicated 150 b.p. fragment was recovered after gel electrophoresis. c) 16 µg of the 1000 b.p. piece was treated with Sau 3a and Ava II. After electrophoresis on a 10 polyacrylamide gel, approximately 0.25 µg (~10 pmole) of the 34 b.p. fragment was recovered. The two indicated deoxyoligonucleotides, 5'-dAATTCATGTGT (fragment 1) and 5'-dGATCACACATG (fragment 2) were synthesized by the phosphotriester procedure (Maxam and Gilbert, Methods Enzymol. 65, 499—560 [1980]). Fragment 2 was phosphorylated as follows. 200 µl (~40 pmole) of $(\gamma^{32}P)$ ATP (Amersham, 5000 Ci/mmole) was dried down and resuspended in 30 µl of 60 mM Tris-HCl (pH 8), 10 mM MgCl$_2$, 15 mM β-mercaptoethanol, containing 100 pmoles of DNA fragment and 2 units of T4 polynucleotide kinase. After 15 minutes at 37°C, 1 µl of 10 mM ATP was added and the reaction allowed to proceed another 15 minutes. The mixture was then heated at 70°C for 15 minutes, combined with 100 pmole of 5'-OH fragment 1 and 10 pmole of the 34 b.p. Sau 3A-Ava II fragment. Ligation was performed for 5 hours at 4°C in 50 µl of 20 mM Tris-HCl (pH 7.5) 10 mM MgCl$_2$, 10 mM dithiothreitol, 0.5 mM ATP and 10 units T4 DNA ligase. The mixture was electrophoresed on a 6 polyacrylamide gel and the 45 b.p. product recovered by electroelution. 860,000 Cerenkov cpm were recovered (~30 ng, 1 pmole), combined with 0.5 µg (5 pmoles) of the 150 b.p. Ava/II-Bgl II fragment and 1 µg (2 pmoles) of the 670 b.p. Bgl II-Pst I fragment. The ligation was performed at 20°C for 16 hours using 20 units of T4 DNA ligase. The ligase was inactivated by heating to 65°C for 10 minutes. The mixture was then digested with Eco RI and Pst I to eliminate polymers of the gene. The mixture was purified by 6 percent polyacrylamide gel electrophoresis. 36,000 cpm (~0.04 pmole, 20 ng) of the 865 b.p. product were isolated. One-half (10 ng) of this was ligated into pBR322 (0.3 µg) between the Eco RI and Pst I sites. Transformation of E. coli 294 gave 70 tetracycline resistant, ampicillin sensitive transformants. Plasmid DNA isolated from 18 of these transformants was digested with Eco RI and Pst I. 16 of the 18 plasmids had an Eco RI-Pst I fragment 865 b.p. in length. One µg of one of these, pLe-IF A1, was digested with Eco RI and ligated to a 300 b.p. Eco RI fragment (0.1 µg) containing the E. coli trp promoter and trp leader ribosome binding site, prepared as described above. Transformants containimg the trp promoter were identified using a $^{32}$P-trp probe in conjunction with the Grunstein-Hogness colony screening procedure—Grunstein et al. [Proc. Natl. Acad. Sci. (USA) 72, 3961 (1975)]. An asymmetrically located Xba I site in the trp fragment allowed determination of recombinants in which the trp promoter was oriented in the direction of the Le-IF A gene.

Extracts were prepared for IF assay as follows. One ml cultures were grown in L broth containing 5 µg/ml tetracycline to an A$_{550}$ of about 1.0, then diluted into 25 ml of M9 media containing 5 µg/ml

6

tetracycline. 10 ml samples were harvested by centrifugation when $A_{550}$ reached 1.0 and cell pellets were suspended in 1 ml of 15 percent sucrose, 50 mM Tris-HCl (pH 8.0), 50 mM EDTA. One mg of lysozyme was added and, after 5 minutes at 0°C, cells were disrupted by sonication. The samples were centrifuged 10 minutes at 15,000 rpm in a Sorvall SM-24 rotor. Interferon activity in the supernatants was determined by comparison with Le-IF standards by the cytopathic effect (CPE) inhibition assay. To determine the number of IF molecules per cell a Le-IF specific activity of $4 \times 10^8$ units/mg was used [Rubinstein et al., Proc. Natl. Acad. Sci. (USA) 76, 640 (1979)].

Clone pLe-IF A trp 25, in which the trp promoter was inserted in the desired orientation, gives high levels of activity (as high as $2.5 \times 10^8$ units per liter). The IF produced by E. coli K-12 strain 294/pLe-IF A trp 25 behaves like authentic human Le-IF; it is stable to treatment at pH2 and is neutralized by rabbit anti-human leukocyte antibodies. The interferon has an apparent molecular weight of approximately 20,000.

Isolation of cDNAs for additional leukocyte interferons

DNA from the fully characterized Le-IF A cDNA-containing plasmid was excised with Pst I, isolated electrophoretically, and labelled by a published procedure [Taylor et al., Biochem. Biophys. Acta. 442, 324 (1976)] with $^{32}P$. The resulting radioactively labelled DNA was used as a probe to screen additional E. coli 294 transformants by an in situ colony screening procedure, Grunstein et al., supra. Colonies were isolated which hybridized in varying amounts to the probe. Plasmid DNA from these colonies and the ten hybridizing colonies referred to above was isolated by Pst cutting and characterized by three different methods. First, these Pst fragments were characterized by their restriction endonuclease digestion patterns with the enzymes Bgl II, Pvu II, and Eco RI. This analysis allowed the classification of at least eight different types (Le-IF A, Le-IF B, Le-IF C, Le-IF D, Le-IF E, Le-IF F, Le-IF G and Le-IF H), which approximates the location of various restriction cuts relative to the by-now known presequence and coding sequence of Le-IF A. One of these, Le-IF D, is believed to be identical to that reported by Nagata et al., Nature 284, 316 (1980).

Secondly, certain of the DNAs were tested by a published hybridization selection assay, Cleveland et al., Cell 20, 95 (1980), for the ability to selectively remove Le-IF mRNA from poly-A containing KG-I cell RNA. Le-IF A, B, C and F were positive by this assay. Third, the latter Pst fragments were inserted in an expression plasmid, E. coli 294 transformed with the plasmid, and the fragments expressed. The expression products, believed to have been preinterferons, were all positive by CPE assay for interferon activity, albeit marginally active in the case of the Le-IF-F fragment. In addition to the foregoing, all of the Le-IF types described have been sequenced.

Second mature leukocyte interferon

The sequence of the isolated fragment comprising the gene for mature Le-IF-B shows the first fourteen nucleotides of types A and B to be identical. We accordingly proposed to isolate a fragment from pLe-IF A25 bearing the trp-promoter-operator, ribosome binding site and the start of the Le-IF (A=B) gene, and combine this with the remaining portion of the B sequence in an expression plasmid.

To obtain the approximately 950 b.p. Sau 3a to Pst I fragment from the sequence shown in Figure 7a several steps were necessary because of the presence of one or more intervening Sau 3a restriction sites, i.e.:

1. The following fragments were isolated:
a) 110 b.p. from Sau 3a to Eco RI;
b) 132 b.p. from Eco RI to Xba;
c) >700 b.p. from Xba to Pst.

2. Fragments (1a) and (1b) were ligated and cut with Xba and Bgl II to preclude self-polymerization through Sau 3a and Xba end terminals (the relevant Sau 3a site was within a Bgl II site; Bgl II cuts to leave a Sau 3a sticky end). A 242 b.p. fragment was isolated.

3. The product of (2) and (1c) were ligated and cut with Pst I and Bgl II, again to prevent self-polymerization. An approximate 950 b.p. fragment, Sau 3a to Pst I of Figure 7a, was isolated. This fragment comprised that portion of the Le-IF B gene not common to Le-IF A.

4. An approximate 300 b.p. fragment (Hind III to Sau 3a) comprising the trp promoter-operator, ribosome binding site, ATG start signal and cysteine codon of Le-IF A was isolated from pLe-IF A25.

5. An approximately 3600 b.p. fragment Pst I to Hind III was isolated from pBr 322. This comprised the replicon and encoded tetracycline but not ampicillin resistance.

6. The fragments obtained in steps 3, 4 and 5 were triple-ligated and the resulting plasmid transformed into E. coli K-12 strain 294.

Transformants were miniscreened, Birnboim et al., Nucleic Acid Research 7, 1513 (1979), and plasmid samples were digested with Eco RI. Digests yielded three fragments characteristic of:

1) The Eco Ri-Eco RI trp promoter fragment; 2) The internal Eco RI to Eco RI fragment of pL4; and 3) protein translational start signal to Eco RI fragment of pL4.

In CPE assay, bacterial extracts from clones made in the foregoing fashion typically assay at about $10 \times 10^6$ units interferon activity per liter at $A_{550}=1$. One representative clone prepared in this manner is 294/pLIF B trp 7.

Further mature leukocyte interferons

Additional full-length gene fragments that comprise other Le-IF types may be tailored and placed in expression vehicles for expression as in the case of Le-IF A. Complete sequencing by conventional means will reveal whether a restriction site lies sufficiently near the first amino acid codon of the mature interferon type as to permit convenient resort to the approach employing the elimination of the presequence by restriction cutting and replacement of codons for the N-terminal amino acids lost in presequence elimination by ligation of a synthetic DNA fragment, as described above. Failing that, the procedure described in Kleid et al., supra, may be employed. Briefly, this entails cleaving the presequence-containing fragment precisely before the point at which the codon for the first amino acid of the mature polypeptide begins, by:

1. converting the double stranded DNA to single-stranded DNA in a region surrounding that point;

2. hybridizing to the single-stranded region formed in step (1) a complementary primer length of single-stranded DNA, the 5' end of the primer lying opposite the nucleotide adjoining the intended cleavage site;

3. restoring that portion of the second strand eliminated in step 1 which lies in the 3' direction from the primer by reaction with DNA polymerase in the presence of adenine, thymine, guanine and cytosine-containing deoxynucleotide triphosphates; and

4. digesting the remaining single-stranded length of DNA which protrudes beyond the intended cleavage point.

A short length of synthetic DNA terminating, at the 3' end of the coding strand, with the translation start signal ATG can then be ligated by, e.g., blunt-end ligation to the resulting tailored gene for the mature interferons and the gene inserted into an expression plasmid and brought under the control of a promoter and its associated ribosome binding site.

In a manner similar to that employed above, gene fragments encoding Le-IF-D were appropriately configured for direct bacterial expression. The expression strategy for this additional leukocyte interferon included resort to the approximately 300 b.p. fragment (Hind III to Sau 3a) comprising the trp promoter-operator, ribosome binding site, ATG start signal and cysteine codon of Le-IF A from pLe-IF A25. To this were combined gene fragments from the Le-IF D coding gene. The resulting plasmid was used to transform E. coli K-12 strain 294. Ligations to form this gene were as follows:

Le-IF D

Isolate from pLe IF-D:

a) 35 b.p. from Sau 3a to AvaII
b) 150 b.p. from Ava II to Bgl II
c) approx. 700 b.p. from Bgl II to Pst I

Isolate from pLe IF A25:

d) 300 b.p. from Hind III to Sau 3a

Isolate from PBr 322:

e) approx. 3600 b.p. from Hind III to Pst I

Construction

(1) ligate (a)+(b), cut with Bgl II and purifiy a 185 b.p. product (1).
(2) ligate (l)+(d), cut with Hind III, Bgl II, and purify the approx. 500 b.p. product (2).
(3) ligate (2)+(c)+(e) and transform E. coli with the resulting plasmid.

A representative clone made in this manner is E. coli K-12 strain 294/pLeIF D trp 11.

The following Table 1 summarises the details for particular constructions of hybrid LeIF plasmids of the present invention.

TABLE 1

| LeIF hybrid | Total amino acids* | Expression plasmid (vector) | Front portion | | Back portion | | Resultant expression plasmid |
|---|---|---|---|---|---|---|---|
| | | | Fragment | Amino acids | Fragment | Amino acids | |
| AD | 165 | a | Xba I-Pvu II of pLeIF A trp 25 (285 bp) | 1—91 | Pvu II-Pst I of pLeIF D trp 11 (~550 bp) | 93—166 | pLeIF AD trp (Pvu II) |
| DA | 166 | a | Xba I-Pvu II of pLeIF D trp 11 (288 bp) | 1—92 | Pvu II-Pst I of pLeIF A trp 25 (~550 bp) | 92—165 | pLeIF DA trp (Pvu II) |
| AD | 165 | — | Bgl II-Pst I large fragment of pLeIF A trp 25 | 1—62 | Bgl II-Pst I of pLeIF D trp II (~600 bp) | 64—166 | pLeIF AD trp (Bgl II) |
| DA | 166 | — | Bgl II-Pst I large fragment pLeIF D trp 11 | 1—63 | Bgl II partial-Pst I of pLeIF A trp 25 (~700 bp) | 63—165 | pLeIF DA trp (Bgl II) |
| AB | 165 | a | Xba I-Pvu II of LeIF A trp 25 (285 bp) | 1—91 | Pvu II partial-Pstl of pLeIF B trp 7 (~750 bp) | 93—166 | pLeIF AB trp (Pvu II) |
| BA | 166 | b | Hind III-Pvu II of pLeIF B trp 7 (~630 bp) | 1—92 | Pvu II-Pst I of pLeIF A trp 25 (~550 bp) | 92—165 | pLeIF BA trp (Pvu II) |
| BD | 166 | b | Hind III-Pvu II of pLeIF B trp 7 (~630 bp) | 1—92 | Pvu II-Pst I of pLeIF D trp 11 (~550 bp) | 93—166 | pLeIF BD trp (Pvu II) |
| DB | 166 | a | Xba I-Pvu II of pLeIF D trp 11 (288 bp) | 1—92 | Pvu II partial-Pst I of pLeIF B trp 7 (~750 bp) | 93—166 | pLeIF DB trp (Pvu II) |

\* excluding N-terminal methionine.
a large (~3900 bp) fragment of Xba I to Pst I digest of pLeIF A trp 25.
b large (~3600 bp) fragment of Hind III to Pst I digest of pBR 322.

With further reference to Table 1, the first four described hybrid LeIFs have been produced from two LeIF expression plasmids. A Bgl II site common to LeIF A and D/cDNAs has been used to construct an expression plasmid pLeIF trp AD (Bgl II) which codes for the 63 amino terminal amino acids of LeIF A and the 102 carboxy terminal amino acids of LeIF D. The same site was utilized in the construction of an expression plasmid pLeIF trp DA (Bgl II) which codes for the 64 amino terminal amino acids of LeIF D and 102 carboxy terminal amino acids of LeIF A. The Pvu II site has been used in the construction of two other hybrid interferon expression plasmids: 91 amino terminal amino acids of A with 74 carboxy terminal amino acids of D (pLeIF trp AD (Pvu II)) and 92 amino terminal amino acids of LeIF D with 74 carboxy terminal amino acids of LeIF A (pLeIF trp DA (Pvu II)). In summary, for:

pLeIF AD trp (Pvu II): The large (~3900 bp) fragment of an Xba I and Pst I digest of pLeIF A trp 25 was ligated with a 285 bp Xba I-Pvu II fragment of pLeIF A trp 25 and an approximately 550 bp Pvu II-Pst I fragment of pLeIF D trp 11;

pLeIF DA trp (Pvu II): The large (~3900 bp) fragment of an Xba I and Pst I digest of pLeIF A trp 25 was ligated with a 288 bp Xba I-Pvu II fragment of pLeIF D trp 11 and an approximately 550 bp Pvu II-Pst I fragment of pLeIF A trp 25;

pLeIF AD trp (Bgl II): The large fragment from a Bgl II, Pst I digest of pLeIF A trp 25 was ligated with a ~600 bp Bgl II-Pst I fragment from pLeIF D trp 11; and

pLeIF DA trp (Bgl II): The large fragment from a Bgl II and Pst I digest of pLeIF D trp 11 was ligated to an approximately 700 bp fragment obtained by Pst I cleavage of pLeIF A trp 25 followed by partial Bgl II digestion.

In the fifth depicted hybrid:

pLeIF AB trp (Pvu II): The large (~3900 bp) fragment of an Xba I and Pst I digest of pLeIF A trp 25 was ligated with a 285 bp Xba I-Pvu II fragment of pLeIF A trp 25 and an approximately 750 bp Pvu II (partial)-Pst I fragment of pLeIF B trp 7.

In like manner, the other constructions depicted in Table 1 are so defined. As a further example, in the construction of a LeIF C and/or LeIF H portion containing hybrid, one can take advantage of common Bbv I sites occurring at about nucleotide 294 (i.e., GCTGC) of the gene sequences.

Preferably, the first fragment mentioned above is derived from an expression plasmid, i.e., one in which codons for the amino terminal portion of the first leukocyte interferon are preceded by an ATG or other translation initiation codon and a promoter or promoter-operator system. As a result, the end product of the manipulative operations described above will be a plasmid capable of expressing the polypeptide encoded by the hybrid gene in bacteria or other microbial organisms transformed with the plasmid. Other means of configuring the hybrid gene for microbial expression will be apparent to those skilled in the art.

In preferred embodiments of the invention, the hybrid genes encode a novel leukocyte interferon amino acid sequence approximating 165—166 amino acids constituting a conjugate of substantial amino acid sequences drawn from two different leukocyte interferons selected from the group consisting of LeIF A, LeIF B, and LeIF D as depicted in Figure 3. Most preferably, the novel luekocyte interferons encoded by the hybrid genes comprise the amino acids specified and positioned as indicated in the sequence "A11" of Figure 3. The expression products of plasmids formed according to the invention may be tested for antiviral activity in conventional manner, as in the biological activity determinations next described.

Demonstration of antiviral activity

E. coli K-12 strain 294 was conventionally transformed with, independently, the plasmids pLeIF trp A 25, pLeIF trp D, pLeIF trp A/D (Bgl II) and pLeIF trp D/A (Bgl II). The transformants were separately grown up in 5 ml cultures in L broth containing 5 mg/ml tetracycline to an $A_{550}$ of about 1.0, then diluted into one liter of M9 media containing 5 µg/ml tetracycline. Cells were harvested when $A_{550}$ reached 1.0 and cell pellets suspended in 10 ml of 15 percent sucrose, 50 mM tris-HCl (pH/8.0), 50 mM EDTA. 10 mg of lysozyme were added and, after 5 minutes at 0°C, cells were disrupted by sonication. The samples were centrifuged 10 minutes at 15,000 rpm in a Sorvall SM-24 rotor. Interferon activity in the supernatants was subjected to test for antiviral activity.

The yields per liter of culture of these interferons, titrated on a human cell line (WISH) are shown in Table 2 from which it is apparent that LeIF-A/D activity is produced in greater amount than the other interferons. This difference could be due to greater intrinsic activity of the LeIF-A/D or to greater yield in terms of mg protein of this interferon. Because the genetic link-up was identical for all these interferons it seems most probable that LeIF-A/D essentially has greater activity than the other interferons.

TABLE 2
Yield of leukocyte interferons from
shaking flask cultures of E. coli

| Type interferon | Activity yield/liter (units on WISH)* |
|---|---|
| A | $8 \times 10^7$ |
| D | $5 \times 10^6$ |
| AD (Bgl II) | $2 \times 10^8$ |
| DA (Bgl II) | $1 \times 10^6$ |

*assayed by inhibition of cytopathic effect on WISH cells with VSV as challenge.

The potency of the various interferons in a range of mammalian cell lines was determined (human, WISH: African green monkey, VERO; hamster fibroblast, BHK; rabbit kidney cells, RK-13; mouse L-929; and bovine kidney, MDBK cells). In order to compare the relative activity of the interferons their activity on various cells was calculated relative to their activity on WISH cells taken as 100. The results in Table 3 show that LeIF-A/D has very high activity in VERO and L-929 cells whereas LeIF-D/A has low activity in these cell lines. These results indicate that the combination of the N-terminal portion of LeIF-A and the C-terminal portion of LeIF-D within one molecule (LeIF-A/D) confers to the hybrid protein particular potency which is manifest in several mammalian species. Moreover, these properties are not simply the summation of the properties of the parent interferons. This is clearly seen in the case of activity on L-929 cells (Table 3) in which case neither a mixture of LeIF-A and LeIF-D nor the other hybrid, LeIF-D/A, has significant activity.

TABLE 3
Titration of various leukocyte interferons in
cell lines from various mammalian species

| Cell line | Leukocyte interferons* | | | | | |
|---|---|---|---|---|---|---|
| | A | D | A/D | D/A | A+D | Buffy-coat |
| WISH | 100 | 100 | 100 | 100 | 100 | 100 |
| VERO | 250 | 75 | 1,670 | 20 | 200 | 200 |
| BHK | 400 | 200 | 833 | 2,000 | 400 | 20 |
| RK-13 | 12 | 500 | 6 | N.D. | N.D. | 120 |
| L-929 | 150 | 5 | 3,300 | 2 | 10 | 0.1 |

*Interferons tested against VSV infection of the different cell lines. Activities expressed as percentage of activity observed in WISH cells.

The activity of LeIF-A/D against other viruses was also examined. The data in Figure 5 show antiviral effects against EMC virus infection of L-cells and the data in Figure 6 shows effects against VSV infection of L-cells. It is clear from these data that the greater activity of LeIF-A/D is not confined to one virus (VSV) and its greater activity is likely to be a general property against many viruses. Natural human buffy-coat interferon preparations have no effect against mouse cells (see Table 2). The activity of LeIF-A/D against EMC virus infection of CD-1 mice was therefore examined. The results in Figure 7 show that LeIF-A/D is extremely potent against lethal EMC virus infection and LeIF-A also has antiviral activity, as is to be expected from the activity in cell lines (Table 2). The data in Figure 7 result from treatments i.p. 3 hrs before infection. Dosages of LeIF-A/D and LeIF-A are as titrated on WISH.

Lethal EMC virus infection of hamsters is also affected by LeIF-A/D and LeIF-A (Figure 8), the former being the most effective, and buffy coat interferon shows only a small and statistically insignificant effect. In the case of Figure 8, all interferons were given i.p. 3 hrs before infection at a dose of $5 \times 10^5$ µg/kg, titrated on WISH cells.

These results indicate that the pronounced antiviral effects of LeIF-A/D in a range of mammalian species is not confined to cell cultures but is also observed in lethal virus infections.

# EP 0 051 873 B1

EMC virus can be considered a model system, a demonstration of antiviral effect against which may be predictive of antiviral effect against the represented family of viruses, e.g., the picornavirus family of which foot and mouth disease and polio are members. VSV virus can be considered a model system, a demonstration of antiviral effect against which may be predictive of antiviral effect against the represented family of viruses, e.g., the rhabdovirus family of which rabies is an important member.

Table 4 tabulates the activities of various of the LeIF hybrids of the present invention on WISH and MDBK cells and the activity ratios thereof:

## TABLE 4

| LeIF hybrid (Pvull) | Units/liter culture WISH cells | Units/liter culture MDBK cells | Ratio activities WISH/MDB |
|---|---|---|---|
| AB | $2.4 \times 10^8$ | $4 \times 10^7$ | 6 |
| AD | $1.2 \times 10^8$ | $2 \times 10^7$ | 6 |
| BA | $1.5 \times 10^7$ | $1 \times 10^7$ | 1.5 |
| BD | $6 \times 10^7$ | $1.5 \times 10^7$ | 4 |
| DA | $3 \times 10^6$ | $1.2 \times 10^8$ | 0.025 |
| DB | $2 \times 10^6$ | $5 \times 10^7$ | 0.04 |
| A* | $8 \times 10^7$ | $1.2 \times 10^8$ | 0.7 |
| B* | $8 \times 10^7$ | $4 \times 10^8$ | 0.2 |
| C* | $2 \times 10^7$ | $1.5 \times 10^7$ | 1.3 |
| D* | $5 \times 10^6$ | $2.5 \times 10^7$ | 0.2 |
| F* | $2 \times 10^7$ | $2 \times 10^8$ | 0.1 |
| I* | $1.6 \times 10^7$ | $1.2 \times 10^7$ | 1.3 |

*For comparison purposes.

Parenteral administration

The hybrid leukocyte interferons hereof may be parenterally administered to subjects requiring antitumor, or antiviral treatment, and to those exhibiting immunosupressive conditions. Dosage and dose rate may parallel that currently in use in clinical investigations of human derived materials, e.g., about $(1—10) \times 10^6$ units daily, and in the case of materials of purity greater than 1 percent, likely up to, e.g., $5 \times 10^7$ units daily. Preliminary indications in the monkey study described above suggest that dosages of bacterially obtained Le-IF could be significantly elevated for greater effect owing to the essential absence of human proteins other than Le-IF, which proteins in leukocyte-derived materials may act as pyrogens, exhibiting adverse effects, e.g., malaise, temperature elevation, etc.

As one example of an appropriate dosage form for essentially homogeneous bacterial Le-IF in parenteral form applicable herein mutatis mutandis, 3 mg Le-IF of specific activity of, say, $2 \times 10^8$ U/mg may be dissolved in 25 ml. 5 N serum albumin (human)-USP, the solution passed through a bacteriological filter and the filtered solution aseptically subdivided into 100 vials, each containing $6 \times 10^6$ units pure interferon suitable for parenteral administration. The vials are preferably stored in the cold ($-20°C$) prior to use.

The compounds of the present invention can be formulated according to known methods to prepare pharmaceutically useful compositions, whereby the polypeptide hereof is combined in admixture with a pharmaceutically acceptable carrier vehicle. Suitable vehicles and their formulation are described in Remington's Pharmaceutical Sciences by E. W. Martin, which is hereby incorporated by reference. Such compositions will contain an effective amount of the interferon protein hereof together with a suitable amount of vehicle in order to prepare pharmaceutically acceptable compositions suitable for effective administration to the host.

**Claims for the Contracting States: BE, CH, DE, FR, GB, IT, LI, LU, NL, SE**

1. An antiviral polypeptide of approximately 165—166 amino acids characterized in that it consists of a

EP 0 051 873 B1

discrete amino acid subsequence of human leukocyte interferon A and of human leukocyte interferon B or D, the overall amino acid sequence of said polypeptide differing from that of naturally occurring leukocyte interferons.

2. A polypeptide according to claim 1 the N-terminal portion thereof consisting of amino acids 1—92 of LeIF-D, viz.

```
C D L P E T H S L D N R R T L M L L A Q M S R I S
P S S C L M D R H D F G F P Q E E F D G N Q F Q K
A P A I S V L H E L I Q Q I F N L F T T K D S S A
A W D E D L L D K F C T E L Y Q Q
```

and the carboxy terminal portion thereof consisting of amino acids 92—165 of LeIF-A, viz.

```
L N D L E A C V I Q G V G V T E T P L M K
E D S I L A V R K Y F Q R I T L Y L K E K K Y S P
C A W E V V R A E I M R S F S L S T N L Q E S L R
S K E.
```

3. A polypeptide according to claim 1 the N-terminal portion thereof consisting of amino acids 1—91 of LeIF-A, viz.

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

and the carboxy terminal portion thereof consisting of amino acids 93—166 of LeIF-D, viz.

```
L N D L E A C V M Q E E R V G E T P L M N
V D S I L A V K K Y F R R I T L Y L T E K K Y S P
C A W E V V R A E I M R S L S F S T N L Q E R L R
R K E.
```

4. A polypeptide according to claim 1 the N-terminal portion thereof consisting of amino acids 1—63 of LeIF-D, viz.

```
C D L P E T H S L D N R R T L M L L A Q M S R I S
P S S C L M D R H D F G F P Q E E F D G N Q F Q K
A P A I S V L H E L I Q Q
```

and the carboxy terminal portion thereof consisting of amino acids 63—165 of LeIF-A, viz.

```
I F N L F S T K D S S A A W D E T L L D K F Y T E
L Y Q Q L N D L E A C V I Q G V G V T E T P L M K
E D S I L A V R K Y F Q R I T L Y L K E K K Y S P
C A W E V V R A E I M R S F S L S T N L Q E S L R
S K E.
```

5. A polypeptide according to claim 1 the N-terminal portion thereof consisting of amino acids 1—62 of LeIF-A, viz.

```
C D L P Q T H S L G S R R T L M L L A Q M R K I S
L F S C L K D R H D F G F P Q E E F G N Q F Q K A
E T I P V L H E M I Q Q
```

and the carboxy terminal portion thereof consisting of amino acids 64—166 of LeIF-D, viz.

```
I F N L F T T K D S S A A W D E D L L D K F C T E
L Y Q Q L N D L E A C V M Q E E R V G E T P L M N
V D S I L A V K K Y F R R I T L Y L T E K K Y S P
C A W E V V R A E I M R S L S F S T N L Q E R L R
R K E.
```

13

6. A polypeptide according to claim 1 the N-terminal portion thereof consisting of amino acids 1—91 of LeIF-A, viz.

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

and the carboxy terminal portion thereof consisting of amino acids 93—166 of LeIF-B, viz.

```
L N D L E V L C D Q E V G V I E S P L M Y E D S I
L A V R K Y F Q R I T L Y L T E K K Y S S C A W E
V V R A E I M R S F S L S I N L Q K R L K S K E.
```

7. A double stranded DNA encoding a polypeptide as claimed in any one of claims 1—5.

8. A double stranded DNA encoding a polypeptide as claimed in claim 6.

9. A replicable plasmidic expression vehicle containing a double stranded DNA as claimed in claim 7 capable, in a transformant bacterium, of expressing a polypeptide as claimed in any one of claims 1—5.

10. A replicable plasmidic expression vehicle containing a double stranded DNA as claimed in claim 8 capable, in a transformant bacterium, of expressing a polypeptide as claimed in claim 6.

11. A transformant bacterium containing an expression vehicle claimed in claim 9.

12. A transformant bacterium containing an expression vehicle as claimed in claim 10.

13. A method of forming a double stranded DNA comprising a sequence encoding a polypeptide as claimed in any one of claims 1—6 which is characterized by

(a) selecting a first, double stranded DNA fragment comprising codons encoding all or a portion of the amino acid sequence of a first, naturally occurring leukocyte interferon, said fragment including codons for the amino terminal portion of said interferon and, spaced in the 3' direction therefrom, a first restriction endonuclease cleavage site;

(b) cleaving said fragment with said restriction endonuclease;

(c) selecting a second double stranded DNA fragment encoding all or a portion of the amino acid sequence of a second, different, naturally occurring leukocyte interferon, the second fragment comprising an identical restriction endonuclease cleavage site essentially identically positioned from the standpoint of the amino acid numbering of the two interferons and additionally comprising, beyond said site, codons for the carboxyl terminal portion of the second interferon;

(d) cleaving the second fragment with said restriction endonuclease; and

(e) ligating restriction endonuclease digestion products of steps b and d to reconstitute said restriction endonuclease site and form a hybrid fragment comprising, in sequence, codons for the amino terminal portion of the first interferon and the carboxy terminal portion of the second.

14. A method of forming an antiviral polypeptide as claimed in any one of claims 1—6 which comprises:

(a) deploying the hybrid DNA fragment resulting from the method claimed in claim 13 within a replicable plasmidic expression vehicle, transforming a bacterium with the same, culturing the transformant and occasioning the expression of the polypeptide encoded by the hybrid DNA fragment; and

(b) lysing the resulting transformant and recovering therefrom the polypeptide.

15. A pharmaceutical composition containing a therapeutically effective amount of a polypeptide as claimed in any one of claims 1—6 and a pharmaceutically acceptable carrier vehicle.

**Claims for the Contracting State: AT**

1. A method of forming a double stranded DNA comprising a sequence encoding an antiviral polypeptide of approximately 165—166 amino acids consisting of a discrete amino acid subsequence of human leukocyte interferon A and of human leukocyte interferon B or D, the overall amino acid sequence of said polypeptide differing from that of naturally occurring leukocyte interferons, which method is characterized by

(a) selecting a first, double stranded DNA fragment comprising codons encoding all or a portion of the amino acid sequence of a first, naturally occurring leukocyte interferon, said fragment including codons for the amino terminal portion of said interferon and, spaced in the 3' direction therefrom, a first restriction endonuclease cleavage site;

(b) cleaving said fragment with said restriction endonuclease;

(c) selecting a second double stranded DNA fragment encoding all or a portion of the amino acid sequence of a second, different, naturally occurring leukocyte interferon, the second fragment comprising an identical restriction endonuclease cleavage site essentially identically positioned from the standpoint of the amino acid numbering of the two interferons and additionally comprising, beyond said site, codons for the carboxyl terminal portion of the second interferon;

(d) cleaving the second fragment with said restriction endonuclease; and

(e) ligating restriction endonuclease digestion products of steps b and d to reconstitute said restriction endonuclease site and form a hybrid fragment comprising, in sequence, codons for the amino terminal portion of the first interferon and the carboxy terminal portion of the second.

2. A method according to claim 1 characterized in that the N-terminal portion of the polypeptide encoded by the DNA consists of amino acids 1—92 of LeIF-D, viz.

```
C D L P E T H S L D N R R T L M L L A Q M S R I S
P S S C L M D R H D F G F P Q E E F D G N Q F Q K
A P A I S V L H E L I Q Q I F N L F T T K D S S A
A W D E D L L D K F C T E L Y Q Q
```

and the carboxy terminal portion thereof consists of amino acids 92—165 of LeIF-A, viz.

```
L N D L E A C V I Q G V G V T E T P L M K
E D S I L A V R K Y F Q R I T L Y L K E K K Y S P
C A W E V V R A E I M R S F S L S T N L Q E S L R
S K E.
```

3. A method according to claim 1 characterized in that the N-terminal portion of the polypeptide encoded by the DNA consists of amino acids 1—91 of LeIF-A, viz.

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

and the carboxy terminal portion thereof consists of amino acids 93—166 of LeIF-D, viz.

```
L N D L E A C V M Q E E R V G E T P L M N
V D S I L A V K K Y F R R I T L Y L T E K K Y S P
C A W E V V R A E I M R S L S F S T N L Q E R L R
R K E.
```

4. A method according to claim 1 characterized in that the N-terminal portion of the polypeptide encoded by the DNA consists of amino acids 1—63 of LeIF-D, viz.

```
C D L P E T H S L D N R R T L M L L A Q M S R I S
P S S C L M D R H D F G F P Q E E F D G N Q F Q K
A P A I S V L H E L I Q Q
```

and the carboxy terminal portion thereof consists of amino acids 63—165 of LeIF-A, viz.

```
I F N L F S T K D S S A A W D E T L L D K F Y T E
L Y Q Q L N D L E A C V I Q G V G V T E T P L M K
E D S I L A V R K Y F Q R I T L Y L K E K K Y S P
C A W E V V R A E I M R S F S L S T N L Q E S L R
S K E.
```

5. A method according to claim 1 characterized in that the N-terminal portion of the polypeptide encoded by the DNA consists of amino acids 1—62 of LeIF-A, viz.

```
C D L P Q T H S L G S R R T L M L L A Q M R K I S
L F S C L K D R H D F G F P Q E E F G N Q F Q K A
E T I P V L H E M I Q Q
```

and the carboxy terminal portion thereof consists of amino acids 64—166 of LeIF-D, viz.

```
I F N L F T T K D S S A A W D E D L L D K F C T E
L Y Q Q L N D L E A C V M Q E E R V G E T P L M N
V D S I L A V K K Y F R R I T L Y L T E K K Y S P
C A W E V V R A E I M R S L S F S T N L Q E R L R
R K E.
```

6. A method according to claim 1 characterized in that the N-terminal portion of the polypeptide encoded by the DNA consists of amino acids 1—91 of LeIF-A, viz.

# EP 0 051 873 B1

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

and the carboxy terminal portion thereof consists of amino acids 93—166 of LeIF-B, viz.

```
L N D L E V L C D Q E V G V I E S P L M Y E D S I
L A V R K Y F Q R I T L Y L T E K K Y S S C A W E
V V R A E I M R S F S L S I N L Q K R L K S K E.
```

7. A method of forming a transformant bacterium capable of expressing an antiviral polypeptide defined in any one of claims 1—5 characterized in that in a manner known per se a bacterium is transformed with a replicable plasmidic expression vehicle containing a double stranded DNA as defined in any one of claims 1—5.

8. A method of forming a transformant bacterium capable of expressing an antiviral polypeptide defined in claim 6 characterized in that in a manner known per se a bacterium is transformed with a replicable plasmidic expression vehicle containing a double stranded DNA as defined in claim 6.

9. A method of forming an antiviral polypeptide as defined in any one of claims 1—6 which comprises:

(a) deploying the hybrid DNA fragment resulting from the method claimed in claim 1 within a replicable plasmidic expression vehicle, transforming a bacterium with the same, culturing the transformant and occasioning the expression of the polypeptide encoded by the hybrid DNA fragment; and

(b) lysing the resulting transformant and recovering therefrom the polypeptide.

10. A process for the preparation of pharmaceutical compositions containing an antiviral polypeptide as defined in any one of claims 1—6 which process comprises mixing said polypeptide with non-toxic, inert, therapeutically compatible carries and bringing the resulting mixture into a suitable pharmaceutical dosage form.

**Patentansprüche für die Vertragsstaaten: BE, CH, DE, FR, GB, IT, LI, LU, NL, SE**

1. Ein antivirales Polypeptid von etwa 165—166 Aminosäuren, dadurch gekennzeichnet, dass es aus einer diskreten Aminosäuresubsequenz von menschlichem Leukozyten-Interferon A und menschlichem Leukozyten-Interferon B oder D besteht, wobei die gesamte Aminosäuresequenz des besagten Polypeptids sich von der natürlich vorkommender Leukozyten-Interferone unterscheidet.

2. Ein Polypeptid gemäss Anspruch 1, dessen N-terminaler Teil aus den Aminosäuren 1—92 des LeIF-D, nämlich:

```
C D L P E T H S L D N R R T L M L L A Q M S R I S
P S S C L M D R H D F G F P Q E E F D G N Q F Q K
A P A I S V L H E L I Q Q I F N L F T T K D S S A
A W D E D L L D K F C T E L Y Q Q
```

besteht und dessen carboxyterminaler Teil aus den Aminosäuren 92—165 des LeIF-A, nämlich:

```
L N D L E A C V I Q G V G V T E T P L M K E D S I L A V
R K Y F Q R I T L Y L K E K K Y S P C A W E V V R A E I
M R S F S L S T N L Q E S L R S K E
```

besteht.

3. Ein Polypeptid gemäss Anspruch 1, dessen N-terminaler Teil aus den Aminosäuren 1—91 des LeIF-A, nämlich

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

besteht und dessen carboxyterminaler Teil aus den Aminosäuren 93—166 des LeIF-D, nämlich:

```
L N D L E A C V M Q E E R V G E T P L M N V D S I L A V
K K Y F R R I T L Y L T E K K Y S P C A W E V V R A E I
M R S L S F S T N L Q E R L R R K E
```

besteht.

16

4. Ein Polypeptid gemäss Anspruch 1, dessen N-terminaler Teil aus den Aminosäuren 1—63 des LeIF-D, nämlich:

```
C D L P E T H S L D N R R T L M L L A Q M S R I S
P S S C L M D R H D F G F P Q E E F D G N Q F Q K
A P A I S V L H E L I Q Q
```

besteht und dessen carboxyterminaler Teil aus den Aminosäuren 63—165 des LeIF-A, nämlich:

```
I F N L F S T K D S S A A W D E T L L D K F Y T E
L Y Q Q L N D L E A C V I Q G V G V T E T P L M K
E D S I L A V R K Y F Q R I T L Y L K E K K Y S P
C A W E V V R A E I M R S F S L S T N L Q E S L R
S K E
```

besteht.

5. Ein Polypeptid gemäss Anspruch 1, dessen N-terminaler Teil aus den Aminosäuren 1—62 des LeIF-A, nämlich:

```
C D L P Q T H S L G S R R T L M L L A Q M R K I S
L F S C L K D R H D F G F P Q E E F G N Q F Q K A
E T I P V L H E M I Q Q
```

besteht und dessen carboxyterminaler Teil aus den Aminosäuren 64—166 des LeIF-D, nämlich:

```
I F N L F T T K D S S A A W D E D L L D K F C T E
L Y Q Q L N D L E A C V M Q E E R V G E T P L M N
V D S I L A V K K Y F R R I T L Y L T E K K Y S P
C A W E V V R A E I M R S L S F S T N L Q E R L R
R K E
```

besteht.

6. Ein Polypeptid gemäss Anspruch 1, dessen N-terminaler Teil aus den Aminosäuren 1—91 des LeIF-A, nämlich:

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

besteht und dessen carboxyterminaler Teil aus den Aminosäuren 93—166 des LeIF-B, nämlich:

```
L N D L E V L C D Q E V G V I E S P L M Y E D S I
L A V R K Y F Q R I T L Y L T E K K Y S S C A W E
V V R A E I M R S F S L S I N L Q K R L K S K E
```

besteht.

7. Eine für ein wie in einem jeden der Ansprüche 1—5 beanspruchtes Polypeptid kodierende, doppelsträngige DNA.

8. Eine für ein wie in Anspruch 6 beanspruchtes Polypeptid kodierende, doppelsträngige DNA.

9. Ein replizierbares plasmidisches Expressionsvehikel, das eine wie in Anspruch 7 beanspruchte, doppelsträngige DNA enthält und in einem transformierten Bakterium ein wie in einem jeden der Ansprüche 1—5 beanspruchtes Polypeptid zu exprimieren vermag.

10. Ein replizierbares plasmidisches Expressionsvehikel, das eine wie in Anspruch 8 beanspruchte, doppelsträngige DNA enthält und in einem transformierten Bakterium ein wie in Anspruch 6 beanspruchtes Polypeptid zu exprimieren vermag.

11. Ein ein in Anspruch 9 beanspruchtes Expressionsvehikel enthaltendes, transformiertes Bakterium.

12. Ein ein in Anspruch 10 beanspruchtes Expressionsvehikel enthaltendes, transformiertes Bakterium.

13. Ein Verfahren zur Herstellung einer doppelsträngigen DNA, die eine für ein wie in einem jeden der Ansprüche 1—6 beanspruchtes Polypeptid kodierende Sequenz umfasst, welches dadurch gekennzeichnet ist, dass:

(a) ein erstes, doppelsträngiges DNA-Fragment ausgewählt wird, das Codons umfasst, die für die gesamte oder einen Teil der Aminosäuresequenz eines ersten, natürlich vorkommenden Leukozyten-Interferons kodieren, wobei besagtes Fragment Codons für den aminoterminalen Teil besagten Interferons und im Abstand davon in 3'-Richtung eine erste Schnittstelle für eine Restriktionsendonuklease einschliesst;

(b) besagtes Fragment mit besagter Restriktionsendonuklease geschnitten wird;

(c) ein zweites, doppelsträngiges DNA-Fragment ausgewählt wird, das für die gesamte oder einen Teil der Aminosäuresequenz eines zweiten, verschiedenen, natürlich vorkommenden Leukozyten-Interferons kodiert, wobei das zweite Fragment eine identische und von Gesichtspunkt der Aminosäurebezifferung der beiden Interferone aus im wesentlichen identisch positionierte Schnittstelle für eine Restriktionsendonuklease umfasst und zusätzlich, über besagte Stelle hinaus, Codons für den carboxyterminalen Teil des zweiten Interferons umfasst;

(d) das zweite Fragment mit besagter Restriktionsendonuklease gespalten wird; und

(e) die Produkte Restriktionsendonukleaseverdauung der Schritte b und d zur Wiederherstellung besagter Restriktionsendonukleasenschnittstelle und zur Bildung eines hybriden Fragmentes, das in der Sequenz Codons für den aminoterminalen Teil des ersten Interferons und den carboxyterminalen Teil des zweiten umfasst, verbunden werden.

14. Ein Verfahren zur Herstellung eines wie in einem jeden der Ansprüche 1—6 beanspruchten, antiviralen Polypeptids, welches umfasst:

(a) die Entwicklung des aus dem in Anspruch 13 beanspruchten Verfahren stammenden, hybriden DNA-Fragmentes innerhalb eines replizierbaren, plasmidischen Expressionsvehikels, die Transformation eines Bakteriums mit demselben, die Kultivierung des Transformanten und die Veranlassung der Expression des von dem hybriden DNA-Fragment kodierten Polypeptids; und

(b) die Lysis des entstandenen Transformanten und die Gewinnung des Polypeptids.

15. Ein pharmazeutisches Präparat, das eine therapeutisch wirksame Menge eines wie in einem jeden der Ansprüche 1—6 beanspruchten Polypeptids und ein pharmazeutische annehmbares Trägermaterial enthält.

**Patentansprüche für den Vertragsstaat: AT**

1. Ein Verfahren zur Herstellung einer doppelsträngigen DNA, die eine für ein antivirales Polypeptid von etwa 165—166 Aminosäuren kodierende Sequenz, die aus einer einzelnen Aminosäuresubsequenz von menschlichem Leukozyten-Interferon A und menschlichem Leukozyten-Interferon B oder D besteht, umfasst und wobei die gesamte Aminosäuresequenz des besagten Polypeptids sich von der natürlich vorkommender Leukozyten-Interferone unterscheidet, wobei das Verfahren dadurch gekennzeichnet ist, dass

(a) ein erstes, doppelsträngiges DNA-Fragment ausgewählt wird, das Codons umfasst, die für die gesamte oder einen Teil der Aminosäuresequenz eines ersten, natürlich vorkommenden Leukozyten-Interferons kodieren, wobei besagtes Fragment Codons für den aminoterminalen Teil besagten Interferons und im Abstand davon in 3'-Richtung eine erste Schnittstelle für eine Restriktionsendonuklease einschliesst;

(b) besagtes Fragment mit besagter Restriktionsendonuklease geschnitten wird;

(c) ein zweites, doppelsträngiges DNA-Fragment ausgewählt wird, das für die gesamte oder einen Teil der Aminosäuresequenz eines zweiten, verschiedenen, natürlich vorkommenden Leukozyten-Interferons kodiert, wobei das zweite Fragment eine identische und von Gesichtspunkt der Aminosäurebezifferung der beiden Interferone aus im wensentlichen identisch positionierte Schnittstelle für eine Restriktionsendonuklease umfasst und zusätzlich, über besagte Stelle hinaus, Codons für den carboxyterminalen Teil des zweiten Interferons umfasst;

(d) das zweite Fragment mit besagter Restriktionsnuklease gespalten wird; und

(e) die Produkte der Restriktionsendonukleaseverdauung der Schritte b und d zur Wiederherstellung besagter Restriktionsenonukleasenschnittstelle und zur Bildung eines hybriden Fragmentes, das in der Sequenz Codons für den aminoterminalen Teil des ersten Interferons und den carboxyterminalen Teil des zweiten umfasst, verbunden werden.

2. Ein Verfahren gemäss Anspruch 1, dadurch gekennzeichnet, dass der durch die DNA kodierte N-terminale Teil des Polypeptids aus den Aminosäuren 1—92 des LeIF-D, nämlich:

```
C D L P E T H S L D N R R T L M L L A Q M S R I S
P S S C L M D R H D F G F P Q E E F D G N Q F Q K
A P A I S V L H E L I Q Q I F N L F T T K D S S A
A W D E D L L D K F C T E L Y Q Q
```

besteht und der carboxyterminale Teil davon aus den Aminosäuren 92—165 des LeIF-A, nämlich

```
L N D L E A C V I Q G V G V T E T P L M K E D S I
L A V R K Y F Q R I T L Y L K E K K Y S P C A W E
V V R A E I M R S F S L S T N L Q E S L R S K E
```

besteht.

18

3. Ein Verfahren gemäss Anspruch 1, dadurch gekennzeichnet, dass der durch die DNA kodierte N-terminale Teil des Polypeptids aus den Aminosäuren 1—91 des LeIF-A, nämlich:

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

besteht und der carboxyterminale Teil davon aus den Aminosäuren 93—166 des LeIF-D, nämlich

```
L N D L E A C V M Q E E R V G E T P L M N V D S I
L A V K K Y F R R I T L Y L T E K K Y S P C A W E V V R
A E I M R S L S F S T N L Q E R L R R K E
```

besteht.

4. Ein Verfahren gemäss Anspruch 1, dadurch gekennzeichnet, dass der durch die DNA kodierte N-terminale Teil des Polypeptids aus den Aminosäuren 1—63 des LeIF-D, nämlich:

```
C D L P E T H S L D N R R T L M L L A Q M S R I S
P S S C L M D R H D F G F P Q E E F D G N Q F Q K
A P A I S V L H E L I Q Q
```

besteht und der carboxyterminale Teil davon aus den Aminosäuren 63—165 des LeIF-A, nämlich:

```
I F N L F S T K D S S A A W D E T L L D K F Y T E
L Y Q Q L N D L E A C V I Q G V G V T E T P L M K
E D S I L A V R K Y F Q R I T L Y L K E K K Y S P
C A W E V V R A E I M R S F S L S T N L Q E S L R
S K E
```

besteht.

5. Ein Verfahren gemäss Anspruch 1, dadurch gekennzeichnet, dass der durch die DNA kodierte N-terminale Teil des Polypeptids aus den Aminosäuren 1—62 des LeIF-A, nämlich:

```
C D L P Q T H S L G S R R T L M L L A Q M R K I S
L F S C L K D R H D F G F P Q E E F G N Q F Q K A
E T I P V L H E M I Q Q
```

besteht und der carboxyterminale Teil davon aus der Aminosäuresequenz 64—166 des LeIF-D, nämlich:

```
I F N L F T T K D S S A A W D E D L L D K F C T E
L Y Q Q L N D L E A C V M Q E E R V G E T P L M N
V D S I L A V K K Y F R R I T L Y L T E K K Y S P
C A W E V V R A E I M R S L S F S T N L Q E R L R
R K E
```

besteht.

6. Ein Verfahren gemäss Anspruch 1, dadurch gekennzeichnet, dass der durch die DNA kodierte N-terminale Teil des Polypeptids aus den Aminosäuren 1—92 des LeIF-A, nämlich:

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

besteht und der carboxyterminale Teil davon aus den Aminosäuren 93—166 des LeIF-B, nämlich:

```
L N D L E V L C D Q E V G V I E S P L M Y E D S I
L A V R K Y F Q R I T L Y L T E K K Y S S C A W E
V V R A E I M R S F S L S I N L Q K R L K S K E
```

besteht.

7. Ein Verfahren zur Herstellung eines transformierten Bakteriums, das zur Expression eines in einem jeden der Ansprüche 1—5 definierten, antiviralen Polypeptids befähigt ist, dadurch gekennzeichnet, dass ein Bakterium mit einem eine wie in einem jeden der Ansprüche 1—5 definierte, doppelsträngige DNA enthaltenden, replizierbaren, plasmidischen Expressionsvehikel in bekannter Weise transformiert wird.

8. Ein Verfahren zur Herstellung eines transformierten Bakteriums, das zur Expression eines in Anspruch 6 definierten, antiviralen Polypeptids befähigt ist, dadurch gekennzeichnet, dass ein Bakterium mit einem eine wie in Anspruch 6 definierte, doppelsträngige DNA enthaltenden, replizierbaren, plasmidischen Expressionsvehikel in bekannter Weise transformiert wird.

9. Ein Verfahren zur Herstellung eines wie in einem jeden der Ansprüche 1—6 definierten, antiviralen Polypeptids, welches umfasst:

(a) die Entwicklung des aus dem in Anspruch 13 beanspruchten Verfahren stammenden, hybriden DNA-Fragmentes innerhalb eines replizierbaren, plasmidischen Expressionsvehikel, die Transformation eines Bakteriums mit demselben, die Kultivierung des Transformanten und die Veranlassung der Expression des von dem hybriden DNA-Fragment kodierten Polypeptids; und

(b) die Lysis des entstandenen Transformanten und die Gewinnung des Polypeptids.

10. Ein Verfahren zur Herstellung von pharmazeutischen Präparaten, die ein wie in einem jeder der Ansprüche 1—6 definiertes, antivirales Polypeptid enthalten, wobei das Verfahren das Mischen besagter Polypeptide mit nicht-toxischen, inerten, therapeutische kompatiblen Trägermaterialien und das Ueberführen der entstandenen Mischung in eine geeignete pharmazeutische Dosierungsform umfasst.

**Revendications pour les Etats Contractants: BE, CH, DE, FR, GB, IT, LI, LU, NL, SE**

1. Polypeptide antiviral d'environ 165—166 acides aminés, caractérisé en ce qu'il se compose d'une sous-séquence discrète d'acides aminés d'interféron A de leucocyte humain et d'interféron B ou D de leucocyte humain, la séquence globale des acides aminés dudit polypeptide différant de celle des interférons de leucocyte que l'on trouve dans la nature.

2. Polypeptide selon la revendication 1, dont la partie N-terminale se compose des acides aminés 1—92 de LeIF-D, à savoir

```
C D L P E T H S L D N R R T L M L L A Q M S R I S
P S S C L M D R H D F G F P Q E E F D G N Q F Q K
A P A I S V L H E L I Q Q I F N L F T T K D S S A
A W D E D L L D K F C T E L Y Q Q
```

et la partie carboxy-terminale se compose des acides aminés 92—165 de LeIF-A, à savoir:

```
L N D L E A C V I Q G V G V T E T P L M K
E D S I L A V R K Y F Q R I T L Y L K E K K Y S P
C A W E V V R A E I M R S F S L S T N L Q E S L R
S K E.
```

3. Polypeptide selon la revendication 1, dont la partie N-terminale se compose des acides aminés 1—91 de LeIF-A, à savoir

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

et dont la partie carboxy-terminale se compose des acides aminés 93—166 de LeIF-D, à savoir

```
L N D L E A C V M Q E E R V G E T P L M N
V D S I L A V K K Y F R R I T L Y L T E K K Y S P
C A W E V V R A E I M R S L S F S T N L Q E R L R
R K E.
```

4. Polypeptide selon la revendication 1, dont la partie N-terminale se compose des acides aminés 1—63 de LeIF-D, à savoir

```
C D L P E T H S L D N R R T L M L L A Q M S R I S
P S S C L M D R H D F G F P Q E E F D G N Q F Q K
A P A I S V L H E L I Q Q
```

et la partie carboxy-terminale se compose des acides aminés 63—165 de LeIF-1, à savoir

```
I F N L F S T K D S S A A W D E T L L D K F Y T E
L Y Q Q L N D L E A C V I Q G V G V T E T P L M K
E D S I L A V R K Y F Q R I T L Y L K E K K Y S P
C A W E V V R A E I M R S F S L S T N L Q E S L R
S K E.
```

5. Polypeptide selon la revendication 1 dont la partie N-terminale se compose des acides aminés 1—62 de LeIF-A, à savoir

```
C D L P Q T H S L G S R R T L M L L A Q M R K I S
L F S C L K D R H D F G F P Q E E F G N Q F Q K A
E T I P V L H E M I Q Q
```

et la partie carboxy-terminale se compose des acides aminés 64—166, de LeIF-D, à savoir

```
I F N L F T T K D S S A A W D E D L L D K F C T E
L Y Q Q L N D L E A C V M Q E E R V G E T P L M N
V D S I L A V K K Y F R R I T L Y L T E K K Y S P
C A W E V V R A E I M R S L S F S T N L Q E R L R
R K E.
```

6. Polypeptide selon la revendication 1, dont la partie N-terminale se compose des acides aminés 1—91, LeIF-A, à savoir

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

et la partie carboxy-terminale se compose des acides aminés 93—166, de LeIF-B, à savoir

```
L N D L E V L C D Q E V G V I E S P L M Y E D S I
L A V R K Y F Q R I T L Y L T E K K Y S S C A W E
V V R A E I M R S F S L S I N L Q K R L K S K E.
```

7. ADN à deux brins encodant un polypeptide selon l'une quelconque des revendications 1—5.

8. ADN à deux brins encodant un polypeptide selon la revendication 6.

9. Véhicule d'expression plasmidique réplicable contenant un ADN à deux brins selon la revendication 7, capable, dans une bactérie transformante, d'exprimer un polypeptide selon l'une quelconque des revendications 1—5.

10. Véhicule d'expression plasmidique réplicable contenant un ADN à deux brins selon la revendication 8, capable, dans une bactérie transformante, d'exprimer un polypeptide selon la revendication 6.

11. Bactérie transformante contenant un véhicule d'expression selon la revendication 9.

12. Bactérie transformante contenant un véhicule d'expression selon la revendication 10.

13. Procédé de formation d'un ADN à deux brins comprenant une séquence encodant un polypeptide selon l'une quelconque des revendications 1—6, qui se caractérise en ce que

(a) on choisit un premier fragment d'ADN à deux brins comprenant des codons encodant la totalité ou une fraction de la séquence d'acides aminés d'un premier interféron de leucocyte que l'on trouve dans la nature, ledit fragment incluant des codons pour la partie amino-terminale dudit interféron et, espacé dans la direction 3', un premier site de clivage d'endonucléase de restriction;

(b) on clive lesdits fragments avec ladite endonucléase de restriction;

(c) on choisit un second fragment d'ADN à deux brins encodant la totalité ou une fraction de la séquence d'acides aminés d'un second interféron de leucocyte, différent, que l'on trouve dans la nature, le second fragment comprenant un site de clivage d'endonucléase de restriction identique positionné de manière essentiellement identique du point de vue de la numérotation des acides aminés des deux interférons et comprenant en outre, au-delà dudit site, des codons pour la partie carboxy-terminale du second interféron;

(d) on clive le second fragment avec ladite endonucléase de restriction; et

(e) on lie les produits de digestion d'endonucléase de restriction des étapes b et d pour reconstituer ledit site d'endonucléase de restriction et former un fragment hybride comprenant, successivement, des codons pour la partie amino-terminale du premier interféron et la partie carboxy-terminale du second.

14. Procédé de formation d'un polypeptide antiviral selon l'une quelconque des revendications 1—6 dans lequel:

(a) on déploie le fragment d'ADN hybride résultant du procédé selon la revendication 13 dans un véhicule d'expression plasmidique réplicable, on l'utilise pour transformer une bactérie, on cultive le trasnformant et on occasionne l'expression du polypeptide encodé par le fragment d'ADN hybride; et

(b) on lyse le transformant résultant et on en recueille le polypeptide.

15. Composition pharmaceutique contenant une quantité thérapeutiquement efficace d'un polypeptide selon l'une quelconque des revendications 1—6 et un véhicule-support parmaceutiquement acceptable.

# EP 0 051 873 B1

**Revendications pour l'Etat Contractant: AT**

1. Procédé de formation d'un ADN à deux brins comprenant une séquence encodant un polypeptide antiviral d'environ 165—166 acides aminés constitué d'une sous-séquence discrète d'acides aminés d'interféron A de leucocyte humain et d'interféron B ou D de leucocyte humain, la séquence globale des acides aminés dudit polypeptide différant de celles des interférons de leucocyte que l'on trouve dans la nature, lequel procédé se caractérise en ce que

(a) on choisit un premier fragment d'ADN à deux brins comprenant des codons encodant la totalité ou une fraction de la séquence d'acides aminés d'un premier interféron de leucocyte que l'on trouve dans la nature, ledit fragment incluant des codons pour la partie amino-terminale dudit interféron et, espacé dans la direction 3', un premier site de clivage d'endonucléase de restriction;

(b) on clive lesdits fragments avec ladite endonucléase de restriction;

(c) on choisit un second fragment d'ADN à deux brins encodant la totalité ou une fraction de la séquence d'acides aminés d'un second interféron de leucocyte, différent, que l'on trouve dans la nature, le second fragment comprenant un site de clivage d'endonucléase de restriction identique positionné de manière essentiellement identique du point de vue de la numérotation des acides aminés des deux interférons et comprenant en outre, au-delà dudit site, des codons pour la partie carboxy-terminale du second interféron.

(d) on clive le second fragment avec ladite endonucléase de restriction; et

(e) on lie les produits de digestion d'endonucléase de restriction des étapes b et d pour reconstituer ledit site d'endonucléase de restriction et former un fragment hybride comprenant, successivement, des codons pour la partie amino-terminale du premier interféron et la partie carboxy-terminale du second.

2. Procédé selon la revendication 1, caractérisé en ce que la partie N-terminale du polypeptide encodé par l'ADN se compose des acides aminés 1—92 de LeIF-D, à savoir

```
C D L P E T H S L D N R R T L M L L A Q M S R I S
P S S C L M D R H D F G F P Q E E F D G N Q F Q K
A P A I S V L H E L I Q Q I F N L F T T K D S S A
A W D E D L L D K F C T E L Y Q Q
```

et sa partie carboxy-terminale se compose des acides aminés 92—165 de LeIF-A, à savoir

```
L N D L E A C V I Q G V G V T E T P L M K
E D S I L A V R K Y F Q R I T L Y L K E K K Y S P
C A W E V V R A E I M R S F S L S T N L Q E S L R
S K E.
```

3. Procédé selon la revendication 1, caractérisé en ce que la partie N-terminale du polypeptide encodé par l'ADN se compose des acides aminés 1—91 de LeIF-A, à savoir

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

et sa partie carboxy-terminale se compose des acides aminés 93—166 de LeIF-D, à savoir

```
L N D L E A C V M Q E E R V G E T P L M N
V D S I L A V K K Y F R R I T L Y L T E K K Y S P
C A W E V V R A E I M R S L S F S T N L Q E R L R
R K E.
```

4. Procédé selon la revendication 1, caractérisé en ce que la partie N-terminale du polypeptide encodé par l'ADN se compose des acides aminés 1—63 de LeIF-D, à savoir

```
C D L P E T H S L D N R R T L M L L A Q M S R I S
P S S C L M D R H D F G F P Q E E F D G N Q F Q K
A P A I S V L H E L I Q Q
```

et sa partie carboxy-terminale se compose des acides aminés 63—165 de LeIF-A, a savoir

```
I F N L F S T K D S S A A W D E T L L D K F Y T E
L Y Q Q L N D L E A C V I Q G V G V T E T P L M K
E D S I L A V R K Y F Q R I T L Y L K E K K Y S P
C A W E V V R A E I M R S F S L S T N L Q E S L R
S K E.
```

5. Procédé selon la revendication 1, caractérisé en ce que la partie N-terminale du polypeptide encodé par l'ADN se compose des acides aminés 1—62 de LeIF-A, à savoir

```
C D L P Q T H S L G S R R T L M L L A Q M R K I S
L F S C L K D R H D F G F P Q E E F G N Q F Q K A
E T I P V L H E M I Q Q
```

et sa partie carboxy-terminale se compose des acides aminés 64—166 de LeIF-D, à savoir

```
I F N L F T T K D S S A A W D E D L L D K F C T E
L Y Q Q L N D L E A C V M Q E E R V G E T P L M N
V D S I L A V K K Y F R R I T L Y L T E K K Y S P
C A W E V V R A E I M R S L S F S T N L Q E R L R
R K E.
```

6. Procédé selon la revendication 1, caractérisé en ce que la partie N-terminale du polypeptide encodé par l'ADN se compose des acides aminés 1—91 de LeIF-A, à savoir

```
C D L P Q T H S L G S R R T L M L L A Q M R K I S
L F S C L K D R H D F G F P Q E E F G N Q F Q K A
E T I P V L H E M I Q Q I F N L F S T K D S S A A
W D E T L L D K F Y T E L Y Q Q
```

et sa partie carboxy-terminale se compose des acides aminés 93—166 de LeIF-B, à savoir

```
L N D L E V L C D Q E V G V I E S P L M Y E D S I
L A V R K Y F Q R I T L Y L T E K K Y S S C A W E
V V R A E I M R S F S L S I N L Q K R L K S K E.
```

7. Procédé de formation d'une bactérie transformante capable d'exprimer un polypeptide antiviral défini dans l'une quelconque des revendications 1—5, caractérisé en ce que de façon connue on transforme une bactérie avec un véhicule d'expression plasmidique réplicable contenant un ADN à deux brins selon l'une quelconque des revendications 1—5.

8. Procédé de formation d'une bactérie transformante capable d'exprimer un polypeptide antiviral défini dans la revendication 6, caractérisé en ce que de façon connue, on transforme une bactérie avec un véhicule d'expression plasmidique réplicable contenant un ADN à deux brins selon la revendication 6.

9. Procédé de formation d'un polypeptide antiviral selon l'une quelconque des revendications 1—6 dans lequel:

(a) on déploie le fragment d'ADN hybride résultant du procédé selon la revendication 1 dans un véhicule d'expression plasmidique réplicable, on l'utilise pour transformer une bactérie, on cultive le transformant et on occasionne l'expression du polypeptide encodé par le fragment d'ADN hybride; et

(b) on lyse le transformant résultant et on en recueille le polypeptide.

10. Procédé de préparation de compositions pharmaceutiques contenant un polypeptide antiviral tel que défini dans l'une quelconque des revendications 1—6, lequel procédé comprend le mélange dudit polypeptide avec des supports non toxiques, inertes, thérapeutiquement acceptables, et la mise du mélange résultant sous une forme posologique pharmaceutique appropriée.

EP 0 051 873 B1

**Fig. 1a**

```
          -60              -40              -20              +1               20               40
LeIF A  TGAGCCTAAACCTTAGGCTCACCCATTTCAACCAGTCTAGCAGCATCTGCAACATCTACAATGGCCTTGACCTTTGCTTTACTGGTGGCCCTCCTGGTGC
LeIF B                             TACTAGCTCAGCAGCATCCGCAACATCTACAATGGCCTTGACTTTTTATTTAATGGTGGCCCTAGTGGTGC

LeIF D          CAAGGTTCAGAGTCACCCATCTCAGCAAGCCCAGAAGTATCTGCAATATCTACGATGGCCTCGCCCTTTGCTTTACTGATGGTCCTGGTGGTGC


                     60              80              100             120              140
LeIF A  TCAGCTGCAAGTCAAGCTGCTCTGTGGGCTGTGATCTGCCTCAAACCCACAGCCTGGGTAGCAGGAGGACCTTGATGCTCCTGGCACAGATGAGGAAAAT
LeIF B  TCAGCTACAAGTCATTCAGCTCTCTGGGCTGTGATCTGCCTCAGACTCACAGCCTGGGTAACAGGAGGGCCTTGATACTCCTGGCACAAATGCGAAGAAT

LeIF D  TCAGCTGCAAGTCAAGCTGCTCTCTGGGCTGTGATCTCCCTGAGACCCACAGCCTGGATAACAGGAGGACCTTGATGCTCCTGGCACAAATGAGCAGAAT


                     160             180             200             220              240
LeIF A  CTCTCTTTTCTCCTGCTTGAAGGACAGACATGACTTTGGATTTCCC CAGGAGGAGTTT    GGCAACCAGTTCCAAAAGGCTGAAACCATCCCTGTCCT
LeIF B  CTCTCCTTTCTCCTGCCTGAAGGACAGACATGACTTTGAATTCCCC CAGGAGGAGTTTGATGATAAACAGTTCCAGAAGGCTCAAGCCATCTCTGTCCT

LeIF D  CTCTCCTTCCTCCTGTCTGATGGACAGACATGACTTTGGATTTCCC CAGGAGGAGTTTGATGGCAACCAGTTCCAGAAGGCTCCAGCCATCTCTGTCCT


                     260             280             300             320              340
LeIF A  CCATGAGATGATCCAGCAGATCTTCAATCTCTTCAGCACAAAGGACTCATCTGCTGCTTGGGATGAGACCCTCCTAGACAAATTCTACACTGAACTCTAC
LeIF B  CCATGAGATGATCCAGCAGACCTTCAACCTCTTCAGCACAAAGGACTCATCTGCTGCTTTGGATGAGACCCTTCTAGATGAATTCTACATCGAACTTGAC

LeIF D  CCATGAGCTGATCCAGCAGATCTTCAACCTCTTTACCACAAAAGATTCATCTGCTGCTTGGGATGAGGACCTCCTAGACAAATTCTGCACCGAACTCTAC


                     360             380             400             420              440
LeIF A  CAGCAGCTGAATGACCTGGAAGCCTGTGTGATACAGGGGGGTGGGGGTGACAGAGACTCCCCTGATGAAGGAGGACTCCATTCTGGCTGTGAGGAAATACT
LeIF B  CAGCAGCTGAATGACCTGGAAGTCCTGTGTGATCAGGAAGTGGGGGGTGATAGAGTCTCCCCTGATGTACGAGGACTCCATCCTGGCTGTGAGGAAATACT

LeIF D  CAGCAGCTGAATGACTTGGAAGCCTGTGTGATGCAGGAGGAGAGGGTGGGAGAAACTCCCCTGATGAATGTGGACTCCATCTTGGCTGTGAAGAAATACT
```

**Fig. 1b**

```
              460           480           500           520           540
              .             .             .             .             .
LeIF A  TCCAAAGAATCACTCTCTATCTGAAAGAGAAGAAATACAGCCCTTGTGCCTGGGAGGTTGTCAGAGCAGAAATCATGAGATCTTTTTCTTTGTCAACAAA
LeIF B  TCCAAAGAATCACTCTATATCTGACAGAGAAGAAATACAGCTCTTGTGCCTGGGAGGTTGTCAGAGCAGAAATCATGAGATCCTTCTCTTTATCAATCAA

LeIF D  TCCGAAGAATCACTCTCTATCTGACAGAGAAGAAATACAGCCCTTGTGCCTGGGAGGTTGTCAGAGCAGAAATCATGAGATCCCTCTCTTTATCAACAAA


              560           580           600           620           640
              .             .             .             .             .
LeIF A  CTTGCAAGAAAGTTTAAGAAGTAAGGAATGAAAACTGGTTCAACATGGAAATGATTTTCATTGATTCGTATGCCAGCTCACCTTTTTATGATCTGCCATT
LeIF B  CTTGCAAAAAAGATTGAAGAGTAAGGAATGAGACCTGGTACAACACGGAAATGATTCTCATAGACTAATACAGCAGTCTACACTTTGACAAGTTGTGCTC

LeIF D  CTTGCAAGAAAGATTAAGGAGGAAGGAATAATATCTGGTCCAACATGAAAACAATTCTTATTGACTCATACACCAGGTCACGCTTTCATGAATTCTGTCA


              660           680           700           720           740
              .             .             .             .             .
LeIF A  TCAAAGACTCATGTTTCTGCTATGACCATGACACGATTTAAATCTTTTCAAATGTTTTTAGGAGTATTAATCAACATTGTATTCAGCTCTTAAGGCACTA
LeIF B  TTTCAAAGACCCTTGTTTCTGCCAAAACCATGCTATGAATTGAATCAAATGTGTCAAGTGTTTTCAGGAGTGTTAAGCAACATCCTGTTCAGCTGTATGG

LeIF D  TTTCAAAGACTCTCACCCCTGCTATAACTATGACCATGCTGATAAACTGATTTATCTATTTAAATATTTATTTAACTATTCATAAGATTTAAATTATTTT


              760           780           800           820           840
              .             .             .             .             .
LeIF A  GTCCCTTACAGAGGACCATGCTGACTGATCCATTATCTATTTAAATATTTTTAAAATATTATTTATTTAACTATTTATAAAACAACTTATTTTTGTTCAT
LeIF B  GCACTAGTCCCTTACAGATGACCATGCTGATGGATCTATTCATCTATTTATTTAAATCTTTATTTAGTTAACTACTATAGGGACTTAAATTAGTTTTGTT

LeIF D  TGTTCATATAACGTCATGTGCACCTTTACACTGTGGTTAGTGTAATAAAACATGTTCCTTATATTTACTC-poly(A)


              860           880           900           920           940
              .             .             .             .             .
LeIF A  ATTACGTCATGTGCACCTTTGCACAGTGGTTAATGTAATAAAAATATGTTCTTTGTATTTGGT-poly(A)
LeIF B  CATATTATATTATGTGAACTTTTACATTGTGAATTGTGTAACAAAAACATGTTCTTATATTTATTATTTTTGCCATGTTTATTAAATTTTTACTATGAAAA


              960           980           1000
              .             .             .
LeIF B  AATTCTTTATTTATTCTTTAAAATTGAACTCCAACCCATGAATTGTGCAAACTGATTAAAGAATGGATGGT-poly(A)
```

EP 0 051 873 B1

base pairs  0     200     400     600     800     1000

**A**    *Pvu* II     *Bgl* II *Pvu* II    *Bgl* II

**B**    *Eco* RI *Eco* RI *Pvu* II    *Pvu* II

**D**    *Pvu* II     *Bgl* II *Pvu* II    *Eco* RI

*FIG. 2.*

```
        S1                  S10                   S20  S23                    10                    20                     30                     40
        •                   •                     •    •                      •                     •                      •                      •
LeIF A  M A L T F A L L V A L L V L S C K S S C S V G C D L P Q T  H S L G S R R T L M L L A Q M R K I S L F S C L K D R H D F G F P Q
LeIF B  M A L T F Y L M V A L V V L S Y K S F S S L G C D L P Q T  H S L G N R R A L I L L A Q M R R I S P F S C L K D R H D F E F P Q

LeIF D  M A S P F A L L M V L V V L S C K S S C S L G C D L P E T  H S L D N R R T L M L L A Q M S R I S P S S C L M D R H D F G F P Q
All     M A     F   L             V L S   K S       S   G C   L       T H S L       R R   L   L       Q M     I S       S C L   D R H D F       P Q

                  50                    60                    70                    80                    90                    100
                  •                     •                     •                     •                     •                     •
LeIF A  E E F - G N Q F Q K A E T I P V L H E M I Q Q I F N L F S  T K D S S A A W D E T L L D K F Y T E L Y Q Q L N D L E A C V I Q G
LeIF B  E E F D D K Q F Q K A Q A I S V L H E M I Q Q T F N L F S  T K D S S A A L D E T L L D E F Y I E L D Q Q L N D L E V L C D Q E

LeIF D  E E F D G N Q F Q K A P A I S V L H E L I Q Q I F N L F T  T K D S S A A W D E D L L D K F C T E L Y Q Q L N D L E A C V M Q E
All     E E F D       Q F Q K A         I   V L H E     Q Q   F N L F     T       S S A             L L       F       E L   Q Q     N D E             Q

              110                   120                   130                   140                   150                   160        166
              •                     •                     •                     •                     •                     •
LeIF A  V G V T E T P L M K E D S I L A V R K Y F Q R I T L Y L K  E K K Y S P C A W E V V R A E I M R S F S L S T N L Q E S L R S K E
LeIF B  V G V I E S P L M Y E D S I L A V R K Y F Q R I T L Y L T  E K K Y S S C A W E V V R A E I M R S F S L S I N L Q K R L K S K E

LeIF D  E R V G E T P L M N V D S I L A V K K Y F R R I T L Y L T  E K K Y S P C A W E V V R A E I M R S L S L S T N L Q E R L R R K E
All           V       P L M       D S I L   V   K Y F   R I T L Y L     E     K Y S   C A W E V V R A E I M R S     S   S         Q     L       K
```

FIG. 3

EP 0 051 873 B1

LeIF A   1

LeIF A    CYS ASP LEU PRO GLN THR HIS SER LEU GLY SER ARG ARG THR LEU MET LEU LEU ALA GLN MET ARG LYS ILE SER LEU PHE SER CYS LEU LYS ASP ARG HIS

LeIF D                      GLU                    ASP ASN                                SER ARG            PRO SER                  MET

LeIF A                                  40                                     50                                     60             BgIII

LeIF A    ASP PHE GLY PHE PRO GLN GLU GLU PHE ···· GLY ASN GLN PHE GLN LYS ALA GLU THR ILE PRO VAL LEU HIS GLU MET ILE GLN GLN│ILE PHE ASN LEU PHE

LeIF D                                    ASP                             PRO ALA       SER              LEU

LeIF A          70                                  80                                90          PvuII                             100

LeIF A    SER THR LYS ASP SER SER ALA ALA TRP ASP GLU THR LEU LEU ASP LYS PHE TYR THR GLU LEU TYR GLN GLN│LEU ASN ASP LEU GLU ALA CYS VAL ILE GLN

LeIF D    THR                                    ASP                     CYS                                             MET

LeIF A                                  110                                   120                                  130

LeIF A    GLY VAL GLY VAL THR GLU THR PRO LEU MET LYS GLU ASP SER ILE LEU ALA VAL ARG LYS TYR PHE GLN ARG ILE THR LEU TYR LEU LYS GLU LYS LYS TYR

LeIF D    GLU GLU ARG       GLY                  ASN VAL                     LYS            ARG                     THR

LeIF A                           140                                  150                              160                      166

LeIF A    SER PRO CYS ALA TRP GLU VAL VAL ARG ALA GLU ILE MET ARG SER PHE SER LEU SER THR ASN LEU GLN GLU SER LEU ARG SER LYS GLU

LeIF D                                           LEU                              ARG          ARG

FIG. 4

EMC VIRUS IN L-CELLS

FIG.5.

TITRATION OF VARIOUS INTERFERONS
AGAINST VS VIRUS IN L-929 CELLS

FIG. 6.

FIG. 7.

EP 0 051 873 B1

LEIF - A/D

LEIF - A

BUFFY COAT

INFECTED
CONTROL

Days Post Infection

Number Hamsters Surviving

*FIG. 8.*

# FIG. 9

```
A                                                                          GTATGTTCCCTA
H                                                                          GTATGTTCCCTA
I                                                                          GTATGTTCCTTA
J                                                                          GTATGTTCACTA
C                                                                          GTATGTTCACTA
```

```
A  TTTAAGGC-TAGGCACAAAGCAAGGTCTTCAGAGAACCTGGAGCCTAAGGTTTAGGCTCACCCATT-TCAACCAGTCTAGCAGCATCTGCAACATCTACA
H  TTTAAGGC-TAGGCACAAAGCAAGGTCTTCAGAGAACCTGGAGCCTAAGGTTTAGGCTCACCCATT-TCAACCAGTCTAGCAGCATCTGCAACATCTACA
I  TTTAAGACCTATGCACAGAGCAAGGTCTTCAGAAAACCTACAACCCAAGGTTCAGTGTTACCCCTCATCAACCAGCCCAGCAGCATCTTCAGGGTTCCCA
J  TTTAAGGCCTATGCACAGAGCAAAGTCTTCAGAAAACCTAGAGGCCAAAGTTCAAGGTTACCCATC-TCAAGTAGCCTAGCAACATTTGCAACATCCCA-
C  TTTAAGACCTATGCACAGAGCAAAGTCTCCAGAAAACCTAGAGGCCACGGTTCAA-GTTACCCACC-TCAGGTAGCCTAGTGATATTTGCAAAATCCCA-
```

```
   +1                                                    .                                          100
A  ATGGCCTTGACCTTTGCTTTACTGGTGGCCCTCCTGGTGCTCAGCTGCAAGTCAAGCTGCTCTGTGGGCTGTGATCTGCCTCAAACCCACAGCCTGGGTA
H  ATGGCATTGCCCTTTGCTTTAATGATGGCCCTGGTGGTGCTCAGCTGCAAGTCAAGCTGCTCTCTGGGCTGTAATCTGTCTCAAACCCACAGCCTGAATA
I  ATGGCCCTGTCCTTTTCTTTACTGATGGCCGTGCTGGTGCTCAGCTACAAATCCATCTGTTCTCTAGGCTGTGATCTGCCTCAGACCCACAGCCTGGGTA
J  ATGGCCCGGTCCTTTTCTTTACTGATGGTCGTGCTGGTACTCAGCTACAAATCCATCTGCTCTCTGGGCTGTGATCTGCCTCAGACCCACAGCCTGCGTA
C  ATGGCCCTGTCCTTTTCTTTACTTATGGCCGTGCTGGTGCTCAGCTACAAATCCATCTGATCTCTGGGCTGTGATCTGCCTCAGACCCACACCCTGCGTA
```

```
                                                                          .                         200
A  GCAGGAGGACCTTGATGCTCCTGGCACAGATGAGGAGAATCTCTCTTTTCTCCTGCTTGAAGGACAGACATGACTTTGGATTTCCCCAGGAGGAGTTT--
H  ACAGGAGGACTTTGATGCTCATGGCACAAATGAGGAGAATCTCTCCTTTCTCCTGCCTGAAGGACAGACATGACTTTGAATTTCCCCAGGAGGAATTTGA
I  ATAGGAGGGCCTTGATACTCCTGGCACAAATGGGAAGAATCTCTCCTTTCTCCTGCCTGAAGGACAGACCTGACTTTGGACTTCCCCAGGAGGAGTTTGA
J  ATAGGAGGGCCTTGATACTCCTGGCACAAATGGGAAGAATCTCTCCTTTCTCCTGCTTGAAGGACAGACATGAATTCAGATTCCCAGAGGAGGAGTTTGA
C  ATAGGAGGGCCTTGATACTCCTGGGACAAATGGGAAGAATCTCTCCTTTCTCCTGCCTGAAGGACAGACATGATTTCCGAATCCCCCAGGAGGAGTTTGA
```

```
                                                                                                    300
A  -GGCAACCAGTTCCAAAAGGCTGAAACCATCCCTGTCCTCCATGAGATGATCCAGCAGATCTTCAATCTCTTCAGCACAAAGGACTCATCTGCTGCTTGG
H  TGGCAACCAGTTCCAGAAAGCTCAAGCCATCTCTGTCCTCCATGAGATGATCCAGCAGACCTTCAATCTCTTCAGCACAGAGAACTCATCTGCTGCTTGG
I  TGGCAACCAGTTCCAGAAGACTCAAGCCATCTCTGTCCTCCATGAGATGATCCAGCAGACCTTCAATCTCTTCAGCACAGAGGACTCATCTGCTGCTTGG
J  TGGCCACCAGTTCCAGAAGACTCAAGCCATCTCTGTCCTCCATGAGATGATCCAGCAGACCTTCAATCTCTTCAGCACAGAGGACTCATCTGCTGCTTGG
C  TGGCAACCAGTTCCAGAAGGCTCAAGCCATCTCTGTCCTCCATGAGATGATCCAGCAGACCTTCAATCTCTTCAGCACAGAGGACTCATCTGCTGCTTGG
```

```
   .                                                                                                400
A  GATGAGACCCTCCTAGACAAATTCTACACTGAACTCTACCAGCAGCTGAATGACCTGGAAGCCTGTGTGATACAGGGGGTGGGGGTGACAGAGACTCCCC
H  GATGAGACCCTCCTAGAAAAAATTCTACATTGAACTTTTCCAGCAAATGAATGACCTGGAAGCCTGTGTGATACAGGAGGTTGGGGTGGAAGAGACTCCCC
I  GAACAGAGCCTCCTAGAAAAAATTTTCCACTGAACTTTACCAGCAACTGAATAACCTGGAAGCATGTGTGATACAGGAGGTTGGGATGGAAGAGACTCCCC
J  GAACAGAGCCTCCTAGAAAAAATTTTCCACTGAACTTTACCAGCAACTGAATGACCTGGAAGCATGTGTGATACAGGAGGTTGGGGTGGAAGAGACTCCCC
C  GAACAGAGCCTCCTAGAAAAAATTTTCCACTGAAATTTACCAGCAACTGAATGACCTGGAAGCATGTGTGATACAGGAGGTTGGGGTGGAAGAGACTCCCC
```

```
                                                                                                    500
A  TGATGAAGGAGGACTCCATTCTGGCTGTGAGGGAAATACTTCCAAAGAATCACTCTCTATCTGAAAGAGAAGAAATACAGCCCTTGTGCCTGGGGAGGTTGT
H  TGATGAATGAGGACTCCATCCTGGCTGTGAAGGAAATACTTCCAAAGAATCACTCTTTATCTGATGGAGAAGAAATACAGCCCTTGTGCCTGGGGAGGTTGT
I  TGATGAATGAGGACTCCATCCTGGCTGTGAGGGAAATACTTCCAAAGAATCACTCTTTATCTAACAGAGAAGAAATACAGCCCTTCAGCCTGGGGAGGTTGT
J  TGATGAATGAGGACTTCATCCTGGCTGTGAGGAAATACTTCCAAAGAATCACTCTTTATCTAATGGAGAAGAAATACAGCCCTTGTGCCTGGGGAGGTTGT
C  TGATGAATGAGGACTCCATCCTGGCTGTGAGGAAATACTTCCAAAGAATCACTCTTTATCTAATAGAGAGGAAATACAGCCCTTGTGCCTGGGAGGTTGT
```

```
                                                                                                    600
A  CAGAGCAGAAATCATGAGATCTTTTTCTTTGTCAACAAACTTGCAAGAAAGTTTAAGAAGTAAGGAATGAAAACTGGTTCAACATGGAAATGATTTTTCAT
H  CAGAGCAGAAATCATGAGATCCCTCTCTTTTTCAACAAACTTGCAAAAAAGATTAAGGAGGAAGGATTGAAAAGTGGTTCATCATGGAAATGATTCTCAT
I  CAGAGCAGAAATCATGAGATCTCTCTCTTTTTCAACAAACTTGCAAAAAATATTAAGGAGGAAGGATTGAAAACTGGTTCAACATGGCAATGATCCTGAT
J  CAGAGCAGAAATCATGAGATCCTTCTCTTTTTCAACAAACTTGAAAAAAGGAGATTAAGGAGGAAGGATTGAAAACTGGTTCATCATGGAAATGATTCTCAT
C  CAGAGCAGAAATCATGAGATCCCTCTCGTTTTCAACAAACTTGCAAAAAAGATTAAGGAGGAAGGATTGAAAACTGGTTCAACATGGCAATGATCCTGAT
```

```
                                                                                                    700
A  TGATTCGTATGCCAGCTCACCTTTTTATGATCTGCCATTTCAAAGACTCATGTTTCTGCTATGACCATGACACGATTTAAATCTTTTCAAATGTTTTTAG
H  TGACTAATACATCATCTCACACTTTCATGAGTTCTTCCATTTCAAAGACTCACTTCTCCTATAACCACCACAAGTTGAATCAAAATTTTCAAATGTTTTC
I  TGACTAATACATTATCTCACACTTTCATGAGTTCCTCCATTTCAAAGACTCACTTCTATAACCACCACGAGTTGAATCAAAATTTTCAAATGTTTTCAGC
J  TGACTAATGCATCATCTCACACTTTCATGAGTTCTTCCATTTCAAAGACTCACTTCTATAACCACCACAAGTTGAATCAAAATTTCCAAATGTTTTCAGG
C  TGACTAATACATTATCTCACACTTTCATGAGTTCTTCCATTTCAAAGACTCACTTCTATAACCACGACGTGTTGAATCAAAATTTTCAAATGTTTTCAGC
```

```
                                                                                                    800
A  GAGTATTAATCAACATTGTATTCAGCTCTTAAGGCACTAGTCCCTTACAGAGGACCATGCTGACTGATCCATTATCTATTTAAATATTTTTAAAATATTA
H  AGGAGTGTAAAGAAGCATCATGTATACCTGTGCAGGCACTAGTCCTTTACAGATGACCATGCTGATGTCTCCTTTCATCTATTTATTTAAATATTTATTT
I  AGTGTAAAGAAGCGTCGTGTATACCTGTGCAGGCACTAGTACTTTACAGATGACCATGCTGATGTCTCTGTTCATCTATTTATTTAAATATTTATTTAAT
J  AGTGTTAAGAAGCATCGTGTTTACCTGTGCAGGCACTAGTCCTTTACAGATGACCATTCTGATGTCTCCTTTCATCTATTTATTTAAATATTTATTTATT
C  AGTGTAAAGAAGTGTCGTGTATACCTGTGCAGGCACTAGTCCTTTACAGATGACCATTCTGATGTCTCTGTTCATCTTTTGTTTAAATATTTATTTAATT
```

```
                                                                                                    900
A  TTTATTTAACTATTTATAAAACAACTTATTTTTGTTCATATTATGTCATGTGCACCTTTGCACAGTGGTTAATGTAATAAAATGTGTTCTTTGTATTTGG
H  ATTTAACTATTTTTATTATTTAAATTATTTTTTATGTTAATATCATGTGTACCTTTACATTGTGGTTAATATAACAAATATGTTCTTCATATTTAGCCAA
I  TATTTTTTAAGATTTAAATTATTTTTTTTATGTAATATCATGTGTACCTTTACATTGTGGTGAATGTAACAATATATGTTCTTCATATTTAGCCAATATATT
J  TAACTATTTTTATTATTTAAATTATTTTTTTATGTAATATCATATGTACCTTTACATTGTGGTTAATGTAACAAATATGTTCTTCATATTTAGCCAATATA
C  ATTTTTAAAATTTATGTAATATCATGAGTCGCTTTACATTGTGGTTAATGTAACAATATATGTTCTTCATATTTAGCCAATATATTAATTTCCTTTTTCA
```

```
                                                                                                    1,000
A  TAAATTTATTTTGTGTTGTTCATTGAACTTTTGCTATGGAACTTTTGTACTTGTTTATTCTTTAAAAATGAAATTCCAAGCCTAATTGTGCAACCTGATTA
H  TATATTAATTTCCTTTTTCATTAAATTTTTACTATACAAAATTTCTGTGTTTGGTATTT
I  AATTTCCTTTTTCATTAAATTTTTTACTATACAAAATTTCTTGTGTGTTTGTTTATTCTTAAGAATAAAATGTCGAGGCTGACTTTACAACCTGACTTAAAAA
J  TTAATTTCCTTTTTCATTAAATTTTTACTATACAAAATTTCTTGTGTTTGTTTATTTTTTAAGATTAAATGCCAAGCCTGACTGTATAACCTGACTTAA
C  TTAAATTTTTTACTATACAAAATTTCTTGTGTTTGTTTATTCTTTAAGATAAAATGCCAAGGCTGACTTTACAACCTGACTTAAAAAATAGATGATTTAATT
```